# EUROPEAN PATENT SPECIFICATION

(11) **EP 4 157 294 B1**
(45) Date of publication and mention of the grant of the patent: **22.07.2026**
(21) Application number: 21734680.8
(22) Date of filing: 28.05.2021
(51) Int. Cl.: A61K 35/30, A61P 27/02, C12N 5/0793, C12N 5/079, G01N 33/50

(54) **RETINAL PIGMENTED EPITHELIUM AND PHOTORECEPTOR DUAL CELL AGGREGATES AND METHODS OF USE THEREOF**
PIGMENTIERTES RETINAEPITHEL- UND PHOTOREZEPTOR-DOPPELZELLAGGREGAT UND VERFAHREN ZUR VERWENDUNG DAVON
AGRÉGATS CELLULAIRES DOUBLES DE PHOTORÉCEPTEURS ET D'ÉPITHÉLIUM PIGMENTÉ RÉTINIEN ET LEURS PROCÉDÉS D'UTILISATION

(30) Priority: 29.05.2020 US 202063032368 P
(43) Date of publication of application: 05.04.2023
(73) Proprietor: FUJIFILM Cellular Dynamics, Inc., Madison, WI 53711 (US)
(72) Inventor: DIAS, Andrew, Madison, WI 53711 (US); BERNDT, Erich, Madison, WI 53711 (US); CHASE, Lucas, Madison, WI 53711 (US)
(74) Representative: Simmons & Simmons LLP (Munich)
(86) International application number: PCT/US2021/034930
(87) International publication number: WO 2021/243256

(56) References cited:
- EP-B1- 2 784 152
- WO-A1-2014/145108
- WO-A1-2017/044483
- WO-A1-2017/044488
- WO-A1-2018/154295
- WO-A1-2019/050015
- WO-A1-2019/204817
- AU-A1- 2014 233 403
- AU-A1- 2016 303 631
- DE-B3- 102017 217 738
- US-A1- 2011 027 333
- US-A1- 2015 125 506
- US-A1- 2017 067 017
- US-A1- 2018 228 846
- US-A1- 2019 169 569
- US-A1- 2020 368 394
- US-A1- 2021 000 102
- US-A1- 2021 238 546
- US-B2- 10 307 444
- US-B2- 10 480 031
- ANONYMOUS: "Evaluation Of Photoreceptor And Retinal Pigment Epithelium Three-dimensional Co-culture Model | IOVS | ARVO Journals", 1 April 2011 (2011-04-01), XP055835826, Retrieved from the Internet <URL:https://iovs.arvojournals.org/article.aspx?articleid=2349980> [retrieved on 20210830]
- WANG XIANMING ET AL: "Generation of a human induced pluripotent stem cell line (HMGUi002-A) from a healthy male individual", STEM CELL RESEARCH, ELSEVIER, NL, vol. 39, 1 August 2019 (2019-08-01), XP085809047, ISSN: 1873-5061, [retrieved on 20190807], DOI: 10.1016/J.SCR.2019.101531
- HIRAMI Y ET AL: "Generation of retinal cells from mouse and human induced pluripotent stem cells", NEUROSCIENCE LETTERS, ELSEVIER, AMSTERDAM, NL, vol. 458, no. 3, 24 July 2009 (2009-07-24), pages 126 - 131, XP026116569, ISSN: 0304-3940, [retrieved on 20090418], DOI: 10.1016/J.NEULET.2009.04.035
- SURENDRAN HARSHINI ET AL: "Transplantation of retinal pigment epithelium and photoreceptors generated concomitantly via small molecule-mediated differentiation rescues visual function in rodent models of retinal degeneration", STEM CELL RESEARCH & THERAPY, vol. 12, no. 1, 1 January 2021 (2021-01-01), XP055835702, Retrieved from the Internet <URL:https://stemcellres.biomedcentral.com/track/pdf/10.1186/s13287-021-02134-x.pdf> DOI: 10.1186/s13287-021-02134-x
- ANONYMOUS: "Comparing Three Methods of Co-culture of Retinal Pigment Epithelium with Progenitor Cells Derived Human Embryonic Stem Cells", 1 November 2013 (2013-11-01), XP055835822, Retrieved from the Internet <URL:https://www.ncbi.nlm.nih.gov/pmc/articles/PMC3883247/?report=printable> [retrieved on 20210830]
- OSAKADA FUMITAKA ET AL: "In vitro differentiation of retinal cells from human pluripotent stem cells by small-molecule induction", JOURNAL OF CELL SCIENCE, COMPANY OF BIOLOGISTS LIMITED, CAMBRIDGE, vol. 122, no. 17, 1 September 2009 (2009-09-01), pages 3169 - 3179, XP002604161, ISSN: 0021-9533, DOI: 10.1242/JCS.050393
- DATABASE BIOSIS [online] BIOSCIENCES INFORMATION SERVICE, PHILADELPHIA, PA, US; May 2011 (2011-05-01), SIMMONS LAUREN ET AL: "Evaluation Of Photoreceptor And Retinal Pigment Epithelium Three-dimensional Co-culture Model", XP002804044, Database accession no. PREV201200525477
- NI Y. ET AL: "ROCK Inhibitor Y-27632 Promotes Human Retinal Pigment Epithelium Survival by Altering Cellular Biomechanical Properties", CURRENT MOLECULAR MEDICINE, vol. 17, no. 9, 9 April 2018 (2018-04-09), NL, pages 637 - 646, XP055835677, ISSN: 1566-5240, Retrieved from the Internet <URL:https://www.ncbi.nlm.nih.gov/pmc/articles/PMC6040175/pdf/CMM-17-637.pdf> DOI: 10.2174/1566524018666180316150936

## Description

### PRIORITY CLAIM

This application claims benefit of priority to U.S. Provisional Application Serial No. 63/032,368, filed May 29, 2020.

### BACKGROUND

### 1. Field

The present disclosure relates generally to the field of stem cell biology. More particularly, it concerns *in vitro* methods as defined in the claims for producing a dual cell aggregate composition comprising retinal epithelial cells (RPE) and photoreceptor precursor cells (PRP), as well as dual cell aggregate compositions obtained with the method for medical use.

### 2. Description of Related Art

Age-related macular degeneration (AMD) is a debilitating condition affecting 11 million people in the United States and 170 million worldwide as of 2016, and global prevalence is expected to reach 196 million in 2020 (Pennington and DeAngelis, 2016; Wong *et al*., 2014). Its cause is presumed dysfunction in the retinal pigmented epithelium (RPE), which leads to death and dysfunction of photoreceptors (Bhutto and Lutty, 2012). Cell therapies using RPE may be effective for treating AMD, myopic macular degeneration, or rarer forms of inherited macular degeneration, and there are currently several ongoing and planned stem cell-based clinical trials to restore visual function (Oner, 2018). While AMD is one of the most common causes of blindness, other dysfunctions, such as retinitis pigmentosa, cone-rod dystrophies, and Leber congenital amaurosis are primarily caused by photoreceptor dysfunction and may be addressable by photoreceptor (PR) transplantation (Barnea-Cramer *et al*., 2016; Zhou *et al*., 2015; Zhao *et al*., 2017).

Photoreceptors extend outer segments that are responsible for light sensing. RPE cells support recycling of shed outer segments and other photoreceptor debris, and support overall photoreceptor health (Strauss, 2005) Therefore, the delivery of both RPE with PR and/or PRP (referred to herein as PR/PRP) as a bilayer culture therapy is a potential opportunity to treat conditions of either RPE or photoreceptor dysfunction, with broader relevant applications than delivery of either cell type alone. Moreover, the symbiotic relationship between RPE and PR/PRP may make such a treatment more effective. Thus, there is an unmet need for a dual cell therapy comprised of PR/PRP and RPE cells for the treatment of these diseases. WO2019/050015 discloses a sphere-like cell aggregate comprising a photoreceptor layer and retinal pigment epithelial cells, for use in therapy. WO2014145108 provides a preparation of photoreceptor progenitor cells for use in the treatment of a disease or disorder caused by loss of photoreceptors. A pharmaceutical preparation is disclosed, comprising retinal pigment epithelial cells and either photoreceptor progenitor cells, photoreceptor cells or both. US2018/228846 discloses a method of treating a retinal disease in a subject by culturing a population of human pluripotent stem cells in a medium and thereby generating a mixed population of cells comprising retinal pigment epithelial (RPE) cells and photoreceptors, generating a photoreceptor-enriched population of cells and administering said population of cells , thereby treating the retinal disease.

### SUMMARY

The present invention is defined in the claims. Subject-matter not encompassed by the claims is disclosed for reference. In certain embodiments, the present disclosure provides an *in vitro* method as defined in the claims for producing a dual cell aggregate composition comprising retinal pigment epithelium cells (RPE) with photoreceptor precursor cells (PRP). In particular aspects, the composition is xeno-free, feeder-free, and defined.

In some aspects, the RPE are mature RPE expressing Bestrophin-1 (BEST1) and/or ZO-1. In certain aspects, the RPE are immature RPE with essentially no expression of BEST1 and/or ZO-1. In certain aspects, the RPE are polarized. In other aspects, the RPE are not polarized.

The composition of the disclosure can be essentially free of a bioabsorbable scaffold and/or extracellular matrix (ECM) proteins. The ratio of PRP to RPE is about 2:1 to about to 50:1, at the time of assembly of the dual cell aggregate composition. In certain aspects, the ratio of PR/PRP to RPE is about 10:1 to about 50:1 at the time of assembly of the dual cell aggregate composition.

In some aspects, the RPE and/or the PRP are derived from pluripotent stem cells (PSCs). In particular aspects, PSCs are induced pluripotent stem cells (iPSCs) or embryonic stem cells (ESCs). For example, the iPSCs are human iPSCs (hiPSCs). In certain aspects, PRP were not derived from organoids. In some aspects, the RPE and/or the PRP have been previously cryopreserved. In specific aspects, the cryopreserved RPE and/or PRP have been thawed and cultured for at least one week.

In particular aspects, the RPE and PRP are formed at a density of about 1 million cells/mL to about 10 million cells/mL, such as about 1, 2, 3, 4, 5, 6, 7, 8, 9, or 10 million cells/mL. In specific aspects, the RPE and PRP are formed at a density of about 5 million cells/mL.

In some aspects, the RPE and/or the PRP are from same donor. In particular aspects, the PRP are rod-predisposed. In some aspects, the PRP are cone-predisposed.

A further embodiment provides a pharmaceutical composition comprising the dual cell aggregate composition of the present embodiments or aspects thereof (e.g., a dual cell aggregate composition comprising RPE with PRP). In some aspects, the dual cell aggregate composition comprises 200,000 to 3,000,000 cells, such as 300,000 to 2,000,000 cells, 400,000 to 1,500,000 cells, 500,000 to 1,000,000 cells, 600,000 to 750,000 cells, or 675,000 to 725,000 cells. In particular aspects, the dual cell aggregate composition comprises about 700,000 cells. In specific aspects, the dual cell aggregate composition comprises 700,000 cells.

In additional aspects, the composition further comprises hyaluronate. In some aspects, the hyaluronate is added at a concentration of less than about 0.5%, such as about 0.4%, 0.3%, 0.2%, or about 0.1%.

In further aspects, the composition further comprises sodium bicarbonate, calcium chloride, potassium chloride, potassium phosphate monobasic, magnesium chloride, magnesium sulfate, sodium chloride, and/or sodium phosphate dibasic. In some aspects, the dual cell aggregate composition is cryopreserved.

The method of the present invention for producing the dual cell aggregate composition or aspects thereof (e.g., a dual cell aggregate composition comprising RPE and PRP) comprises seeding RPE and PRP in culture medium comprising the ROCK inhibitor Y-27632 and culturing for a period of time sufficient to produce the dual cell aggregate composition.

In some aspects, the RPE and PRP are seeded as essentially single-cell suspensions. In other aspects, the RPE are seeded as an essentially single-cell suspension and the PRP are seeded as aggregates.

The ROCK inhibitor is Y-27632. In specific aspects, the Y-27632 is added at a concentration of 10 µM.

The ratio of PRP to RPE is about 2:1 to about 50:1, at the time of assembly of the dual cell aggregate composition.

In further aspects, the culture medium further comprises prostaglandin E2 (PGE-2). In some aspects, the RPE were previously cultured in the presence of PGE-2.

In some aspects, the PRP express PRPH2. In certain aspects, the PRP are rod-disposed. In some aspects, the PRP are cone-disposed.

In certain aspects, the RPE and PRP are seeded at a density of about 1 million cells/mL to about 10 million cells/mL, such as about 1, 2, 3, 4, 5, 6, 7, 8, 9, or 10 million cells/mL. In particular aspects, the RPE and PRP are seeded at a density of about 5 million cells/mL. In some aspects, the RPE and/or the PRP have been previously cryopreserved.

In additional aspects, the culture medium further comprises taurine and hydrocortisone. In some aspects, the culture medium further comprises triiodothyronine. In particular aspects, the culture medium is defined media or serum-free media. In specific aspects, the culture medium comprises serum replacement. In some aspects, culture medium is RPE-MM media.

In some aspects, the culturing is for at least 10 days, such as for at least 2 weeks, 3 weeks, 1 month, or two months. In certain aspects, the culture medium is exchanged at least once every five days, such as at least once every four days, three days, or every other day.

In further aspects, the method further comprises cryopreserving the dual cell aggregate composition.

Another embodiment provides the dual cell aggregate composition produced by the method of the invention for use in a method for treating an ocular injury or disorder in a subject comprising transplanting an effective amount of the dual cell aggregate composition of the present embodiments or aspects thereof (e.g., a dual cell aggregate composition comprising RPE and PRP) to an eye of the subject.

In some aspects, the dual cell aggregate composition is administered at a dose of 200,000 to 3,000,000 cells, such as 300,000 to 2,000,000 cells, 400,000 to 1,500,000 cells, 500,000 to 1,000,000 cells, 600,000 to 750,000 cells, or 675,000 to 725,000 cells. In particular aspects, the dual cell aggregate composition is administered at a dose of about 700,000 cells. In specific aspects, the dual cell aggregate composition is administered at a dose of 700,000 cells. In some aspects, the subject is administered the dual cell aggregate more than once.

In some aspects, the composition is transplanted to the subretinal space of the eye. In certain aspects, the ocular disorder is due to RPE dysfunction or photoreceptor dysfunction. For example, the ocular disorder is age-related macular degeneration, retinitis pigmentosa, cone-rod dystrophies, or Leber congenital amaurosis. In particular aspects, the dual cell aggregate composition produces both rod and cone photoreceptors in the eye of the subject.

Further provided herein is the *ex vivo* use of the dual cell aggregate composition produced by the method of the present embodiments or aspects thereof (e.g., a dual cell aggregate composition comprising RPE and PRP) as a model retina.

A further embodiment provides an *in vitro* method of screening a compound comprising contacting one or more candidate compounds with the dual cell aggregate composition produced by the method of the present invention or aspects thereof (e.g., a dual cell aggregate composition comprising RPE and PRP) and detecting an effect on the RPE-PRP dual cell aggregates.

In some aspects, the one or more candidate compounds are selected from the group consisting of a chemical compound, small molecule, polypeptide, growth factor, solvent, oligonucleotide, and cytokine. In certain aspects, detecting an effect comprises measuring cell proliferation, cell viability, cell death, drug toxicity, or retinal tissue maintenance or repair. The method is *in vitro.* In some aspects, the method is performed in a high-throughput manner. In particular aspects, the dual cell aggregate composition is placed in a multi-well culture plate.

In yet another embodiment, there is provided an *in vitro* retina model comprising the dual cell aggregate composition produced by the method of the present embodiments or aspects thereof (e.g., a dual cell aggregate composition comprising RPE and PRP).

In some aspects, the RPE and/or PRP are obtained from a disease cell line. In particular aspects, the disease is an ocular disease, such as age-related macular degeneration, retinitis pigmentosa, cone-rod dystrophies, or Leber congenital amaurosis.

Other objects, features and advantages of the present disclosure will become apparent from the following detailed description. It should be understood, however, that the detailed description and the specific examples, while indicating preferred embodiments of the invention, are given by way of illustration only..

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein. Subject-matter of the drawings not encompassed by the claims is disclosed for reference.
**FIGS. 1A-1C****:** Dual cell aggregates **(****FIG. 1A****)** 2 days, **(****FIG. 1B****)** 21 days, and **(****FIG. 1C****)** 69 days after aggregate formation in RPE-MM without Y-27632 at a ratio of 1 RPE : 3 PRP.
**FIGS. 2A-2C****:** Dual cell aggregates **(****FIG. 2A****)** 1 day, **(****FIG. 2B****)** 3 days, and (FIG. 2C) 45 days after aggregate formation in RPE-MM with Y-27632 at a ratio of 1 RPE : 8 PRP.
**FIG. 3****:** Dual cell aggregates one month after aggregate formation (initial ratio 1 RPE : 8 PRP), with and without addition of PGE2. Differences in PRP organization were apparent, possibly with more PRP restriction to the aggregate periphery without PGE2.
**FIG. 4****:** One month of dual cell aggregate culture with and without addition of PGE2 did not show striking differences in RPE organization or rod formation, and PRPH2 expression appeared low or absent at this time point.
**FIG. 5****:** Comparison of dual cell aggregate culture with addition of PGE2 1, 2, and 3 months after aggregate formation. Presence of PRPH2 after 2 months indicates outer segment formation and photoreceptor maturation.
**FIG. 6****:** Flow cytometry evaluation of dual cell aggregates two days after aggregate formation. Aggregates were formed with an input ratio of 1 RPE : 30 PRP. Measured RPE:PRP ratio after two days is approximately 1:20, using the RPE markers PMEL and TYRP1 and the PRP marker recoverin.
**FIG. 7****:** Phase contrast images of dual cell aggregates in extended culture in vitro. Pigmented RPE was visible after 9 days, and RPE regions appeared to grow over time.
**FIG. 8****:** Immunocytochemistry of dual cell aggregates at two days showing all cells with Hoechst 33342. RPE (PMEL, TYRP1) occupies a small fraction of the aggregates, while PRP (NRL, CRX) is a large fraction of the aggregates at this time point.
**FIGS. 9A-9F****:** Immunocytochemistry of dual cell aggregates after one month of culture *in vitro.* RPE (**(****FIGS.** 9**A-9B****)** ZO-1, **(****FIGS. 9C-**9**D****)** PMEL, **(****FIGS. 9E-**9**F****)** TYRP1) appears as a larger fraction of the aggregates than at two days, but PRP (recoverin, M/L opsin, NRL, CRX, ARR3) is still the dominant fraction. Presence of both cone (M/L opsin, ARR3) and rod (NRL) markers confirms that PRP can form both cell types, and early presence of PRPH2 indicates the beginning of outer segment markers and suggests PRP are starting to mature.

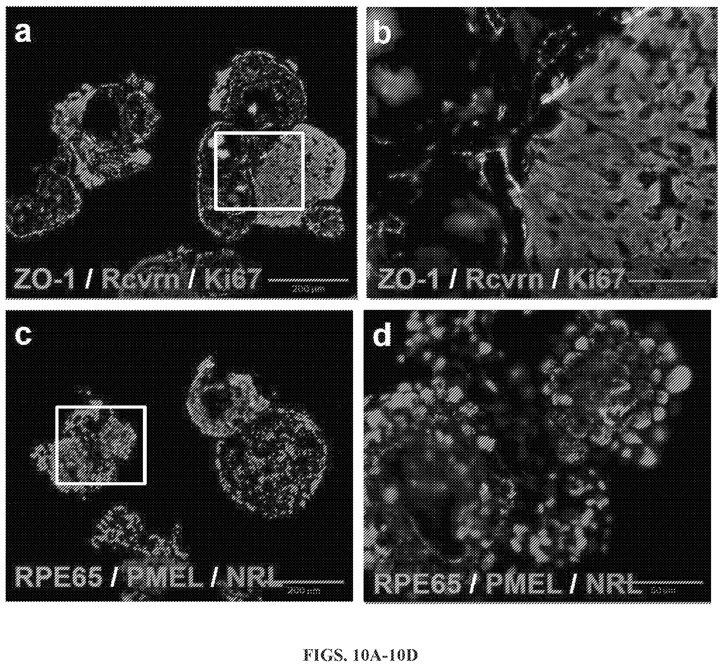

**FIGS. 10A-10D****:** RPE/PRP co-aggregates 3 months after aggregate formation with a 1:30 RPE:PRP ratio at aggregate formation. PRP and RPE form distinct segments in the aggregate shown **(****FIGS. 10A-10B****)** ZO-1 (green) indicating RPE and RCVRN (red) indicating PRP. Proliferating cells (purple) generally appear absent at 3 months. B) shows magnification of inset in A). Maturation of RPE **(****FIG. 10C****)** is shown by RPE65 (green) in a region of PMEL-positive (red) RPE, with rod-fated PRP shown by NRL (purple). **(****FIG. 10D****)** shows magnified inset in **(****FIG. 10C****).**
**FIG. 11****:** Flow cytometry plots of RPE/PRP co-aggregates 3 months after aggregate formation with a 1:30 RPE:PRP ratio at aggregate formation. Plotting both RPE (PMEL) and PRP (RCVRN) shows that there are more RPE than PRP cells at this time point by almost a 2:1 ratio.

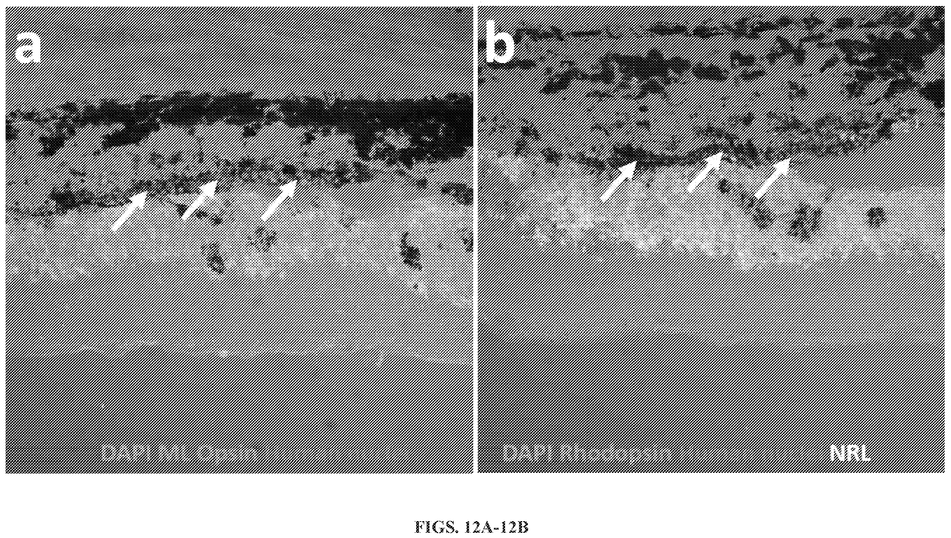

**FIGS. 12A-12B****:** Sections of rat retina one month after dual cell aggregate injection (Condition A; PRP and RPE combined as single-cell suspensions and permitted to form aggregates). Pigmented human cells (black/red) appeared to migrate to the RPE layer in vivo, and there is evidence of both **(****FIG. 12A****)** cone (M/L opsin) and **(****FIG. 12B****)** rod (rhodopsin, NRL) photoreceptors from transplanted cells. Some small clusters of pigmented cells are apparent within the PRP layer.
**FIGS. 13A-13C****:** Sections of rat retina two months after dual cell aggregate injection (Condition A). There is evidence of both rod and cone maturation, indicated by **(****FIG. 13A****)** NRL, **(****FIG. 13B****)** M/L opsin and **(****FIG. 13C****)** rhodopsin in photoreceptors from transplanted cells. Some small clusters of pigmented cells are apparent within the PRP layer.
**FIGS. 14A-14C****:** Sections of rat retina two months after dual cell aggregate injection (Condition B). There is evidence of both rod and cone maturation, indicated by **(****FIG. 14A****)** NRL, **(****FIG. 14B****)** M/L opsin and **(****FIG. 14C****)** rhodopsin in photoreceptors from transplanted cells. Some small clusters of pigmented cells are apparent within the PRP layer.

### I. Description of Illustrative Embodiments

RPE and the neural retina develop and exist in an ordered layer *in vivo.* Recapitulation of native RPE:PRP structural relationship may be essential in a therapy. Moreover, *in vitro* recapitulation of RPE:PRP structure may be viable as a platform to test drugs or cellular disease models. Thus, in certain embodiments, the present disclosure provides methods for culturing human induced pluripotent stem cell (hiPSC)-derived RPE (iRPE) and hiPSC-derived PRP (iPRP) in a "dual-therapy" co-aggregate culture.

The RPE may be derived from hiPSCs, such as by the method disclosed in PCT/US2016/050543 and PCT/US2016/050554. The PRP may also be derived from hiPSCs, such as by the method disclosed in PCT/US2019/028557. The RPE, or PRP may be derived from PSCs, such as embryonic stem cells. PRPs that are immature with a capacity to mature into rods and cones are added to RPE either as single cells or as aggregates. The PRP may be thawed and directly seeded with the RPE, or may be cultured for a period of time prior to seeding with the RPE. The ROCK inhibitor Y-27632 is added to the culture system to supplement RPE-PRP aggregate formation).

The RPE-PRP dual cell aggregate provided herein may be used in a variety of *in vivo* and *in vitro* methods. The RPE-PRP dual cell aggregate may be used *in vitro* in screening assays to identify putative therapeutic or prophylactic treatment candidates. The RPE-PRP dual cell aggregates may be used *in vivo* to treat conditions of the retina, including but not limited to age-related or inherited macular degeneration and retinitis pigmentosa or other inherited outer retinal degenerative diseases or injuries causing dysfunction and/or loss of RPE and/or PRP. Further embodiments and advantages of the present disclosure are described below.

### I. Definitions

The term "purified" does not require absolute purity; rather, it is intended as a relative term. Thus, a purified population of cells is greater than about 90% 91%, 92%, 93%, 94%, 95%, 96%, 97%, 98%, 99% or 100% pure, or, most preferably, essentially free of other cell types.

As used herein in the specification, "a" or "an" may mean one or more. As used herein in the claim(s), when used in conjunction with the word "comprising," the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

The term "essentially" is to be understood that methods or compositions include only the specified steps or materials and those that do not materially affect the basic and novel characteristics of those methods and compositions.

As used herein, a composition or media that is "substantially free" of a specified substance or material contains ≤ 30%, ≤ 20%, ≤ 15%, more preferably ≤ 10%, even more preferably ≤ 5%, or most preferably ≤ 1% of the substance or material.

The terms "substantially" or "approximately" as used herein may be applied to modify any quantitative comparison, value, measurement, or other representation that could permissibly vary without resulting in a change in the basic function to which it is related.

The term "about" means, in general, within a standard deviation of the stated value as determined using a standard analytical technique for measuring the stated value. The terms can also be used by referring to plus or minus 5% of the stated value.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

The term "cell population" is used herein to refer to a group of cells, typically of a common type. The cell population can be derived from a common progenitor or may comprise more than one cell type. An "enriched" cell population refers to a cell population derived from a starting cell population (e.g., an unfractionated, heterogeneous cell population) that contains a greater percentage of a specific cell type than the percentage of that cell type in the starting population. The cell populations may be enriched for one or more cell types and depleted of one or more cell types.

The term "stem cell" refers herein to a cell that under suitable conditions is capable of differentiating into a diverse range of specialized cell types, while under other suitable conditions is capable of self-renewing and remaining in an essentially undifferentiated pluripotent state. The term "stem cell" also encompasses a pluripotent cell, multipotent cell, precursor cell and progenitor cell. Exemplary human stem cells can be obtained from hematopoietic or mesenchymal stem cells obtained from bone marrow tissue. Exemplary pluripotent stem cells can also be produced from somatic cells by reprogramming them to a pluripotent state by the expression of certain transcription factors associated with pluripotency; these cells are called "induced pluripotent stem cells" or "iPSCs".

The term "pluripotent" refers to the property of a cell to differentiate into all other cell types in an organism, with the exception of extraembryonic, or placental, cells. Pluripotent stem cells are capable of differentiating to cell types of all three germ layers (e.g., ectodermal, mesodermal, and endodermal cell types) even after prolonged culture. A pluripotent stem cell is an embryonic stem cell derived from the inner cell mass of a blastocyst. In other embodiments, the pluripotent stem cell is an induced pluripotent stem cell derived by reprogramming somatic cells.

The term "differentiation" refers to the process by which an unspecialized cell becomes a more specialized type with changes in structural and/or functional properties. The mature cell typically has altered cellular structure and tissue-specific proteins.

As used herein, "undifferentiated" refers to cells that display characteristic markers and morphological characteristics of undifferentiated cells that clearly distinguish them from terminally differentiated cells of embryo or adult origin.

"Embryoid bodies (EBs)" are aggregates of pluripotent stem cells that can undergo differentiation into cells of the endoderm, mesoderm, and ectoderm germ layers. The spheroid structures form when pluripotent stem cells are allowed to aggregate under non-adherent culture conditions and thus form EBs in suspension.

An "isolated" cell has been substantially separated or purified from others cells in an organism or culture. Isolated cells can be, for example, at least 99%, at least 98% pure, at least 95% pure or at least 90% pure.

An "embryo" refers to a cellular mass obtained by one or more divisions of a zygote or an activated oocyte with an artificially reprogrammed nucleus.

An "embryonic stem (ES) cell" is an undifferentiated pluripotent cell which is produced by artificial means (*e.g.* nuclear transfer) and can give rise to any differentiated cell type in an embryo or an adult, including germ cells (*e.g.* sperm and eggs).

"Induced pluripotent stem cells (iPSCs)" are cells generated by reprogramming a somatic cell by expressing or inducing expression of a combination of factors (herein referred to as reprogramming factors). iPSCs can be generated using fetal, postnatal, newborn, juvenile, or adult somatic cells. In certain embodiments, factors that can be used to reprogram somatic cells to pluripotent stem cells include, for example, Oct4 (sometimes referred to as Oct 3/4), Sox2, c-Myc, and Klf4, Nanog, and Lin28. In some embodiments, somatic cells are reprogrammed by expressing at least two reprogramming factors, at least three reprogramming factors, or four reprogramming factors to reprogram a somatic cell to a pluripotent stem cell.

An "allele" refers to one of two or more forms of a gene. Diploid organisms such as humans contain two copies of each chromosome, and thus carry one allele on each.

The term "homozygous" is defined as containing two of the same alleles at a particular locus. The term "heterozygous" refers to as containing two different alleles at a particular locus.

A "haplotype" refers to a combination of alleles at multiple loci along a single chromosome. A haplotype can be based upon a set of single-nucleotide polymorphisms (SNPs) on a single chromosome and/or the alleles in the major histocompatibility complex.

As used herein, the term "haplotype-matched" is defined as the cell (*e.g.* iPS cell) and the subject being treated share one or more major histocompatibility locus haplotypes. The haplotype of the subject can be readily determined using assays well known in the art. The haplotype-matched iPS cell can be autologous or allogeneic. The autologous cells which are grown in tissue culture and differentiated to PRP cells inherently are haplotype-matched to the subject.

"Substantially the same HLA type" indicates that the Human Leukocyte Antigen (HLA) type of donor matches with that of a patient to the extent that the transplanted cells, which have been obtained by inducing differentiation of iPSCs derived from the donor's somatic cells, can be engrafted when they are transplanted to the patient.

"Super donors" are referred to herein as individuals that are homozygous for certain MHC class I and II genes. These homozygous individuals can serve as super donors and their cells, including tissues and other materials comprising their cells, can be transplanted in individuals that are either homozygous or heterozygous for that haplotype. The super donor can be homozygous for the HLA-A, HLA-B, HLA-C, HLA-DR, HLA-DP or HLA-DQ locus/loci alleles, respectively.

"Feeder-free" or "feeder-independent" is used herein to refer to a culture supplemented with cytokines and growth factors (*e.g.,* TGFβ, bFGF, LIF) as a replacement for the feeder cell layer. Thus, "feeder-free" or feeder-independent culture systems and media may be used to culture and maintain pluripotent cells in an undifferentiated and proliferative state. In some cases, feeder-free cultures utilize an animal-based matrix (*e.g.* MATRIGEL^{™}) or are grown on a substrate such as fibronectin, collagen, or vitronectin. These approaches allow human stem cells to remain in an essentially undifferentiated state without the need for mouse fibroblast "feeder layers."

"Feeder layers" are defined herein as a coating layer of cells such as on the bottom of a culture dish. The feeder cells can release nutrients into the culture medium and provide a surface to which other cells, such as pluripotent stem cells, can attach.

The term "defined" or "fully-defined," when used in relation to a medium, an extracellular matrix, or a culture condition, refers to a medium, an extracellular matrix, or a culture condition in which the chemical composition and amounts of approximately all the components are known. For example, a defined medium does not contain undefined factors such as in fetal bovine serum, bovine serum albumin or human serum albumin. Generally, a defined medium comprises a basal media (*e.g.,* Dulbecco's Modified Eagle's Medium (DMEM), F12, or Roswell Park Memorial Institute Medium (RPMI) 1640, containing amino acids, vitamins, inorganic salts, buffers, antioxidants, and energy sources) which is supplemented with recombinant albumin, chemically defined lipids, and recombinant insulin. An example of a fully defined medium is Essential 8^{™} medium.

For a medium, extracellular matrix, or culture system used with human cells, the term "Xeno-Free (XF)" refers to a condition in which the materials used are not of non-human animal-origin.

"Pre-confluent" refers to a cell culture in which the proportion of the culture surface which is covered by cells is about 60-80%. Usually, pre-confluent refers to a culture in which about 70% of the culture surface is covered by cells.

The term "retinal progenitor cells", also called "retinal precursor cells" or "RPCs", encompass cells which are competent for generating all cell types of the retina, including neural retina cells, such as rods, cones, photoreceptor precursor cells, as well as cells which can differentiate into RPE.

The term "neural retinal progenitors" or "NRPs" refers to cells which are restricted in their differentiation potential to neural retina cell types.

The term "photoreceptor" or "PR" cells refer to cells that are within the photoreceptor lineage (i.e., maturation) pathway, both before and after upregulation of expression of rhodopsin (rods) or any of the three cone opsins (cones), which encompasses both early and late markers of photoreceptor cells (rod, cone or both).

The terms "photoreceptor precursor cells" or "PRP" refer to cells differentiated from embryonic stem cells or induced pluripotent stem cells which can differentiate into photoreceptor cells that expresses the cell marker rhodopsin or any of the three cone opsins. The photoreceptors may be rod and/or cone photoreceptors.

"Retinal pigment epithelium" refers to a layer of pigmented cells between the choroid, a layer filled with blood vessels, and the neural retina.

The term "retinal degeneration-related disease" is intended to refer to any disease resulting from innate or postnatal retinal degeneration or abnormalities. Examples of retinal degeneration-related diseases include retinal dysplasia, retinal degeneration, age-related macular degeneration, Stargardt disease, Best disease, choroideremia, inherited macular degeneration, myopic degeneration, RPE tears, macular hole, diabetic retinopathy, retinitis pigmentosa, inherited retinal disease or degeneration, inherited macular degeneration, cone-rod dystrophy, rod-cone dystrophy, congenital retinal dystrophy, Leber congenital amaurosis, retinal detachment, and retinal trauma.

A "therapeutically effective amount" used herein refers to the amount of a compound that, when administered to a subject for treatment of a disease or condition, is sufficient to affect such treatment.

"Mature" RPE cells are referred to herein as RPE cells which have downregulated expression of immature RPE markers such as Pax6 and upregulated expression of mature RPE markers such as RPE65.

RPE cell "maturation" refers herein to the process by which RPE developmental pathways are modulated to generate mature RPE cells. For example, modulation of cilia function can result in RPE maturation.

"Inducer" is defined herein as a molecule that regulates gene expression such as activating genes within a cell. An inducer can bind to repressors or activators. Inducers functions by disabling repressors.

As used herein, the term "biodegradable" refers to a material that provides initial structural support to delivered cells, but degrades over time into products that are not toxic to the transplant host and do not contribute to donor site morbidity.

### II. Induced Pluripotent Stem Cells

The induction of pluripotency was originally achieved in 2006 using mouse cells (Yamanaka *et al*. 2006) and in 2007 using human cells (Yu *et al*. 2007; Takahashi *et al*. 2007) by reprogramming of somatic cells via the introduction of transcription factors that are linked to pluripotency. Pluripotent stem cells can be maintained in an undifferentiated state and can differentiate into any adult cell type.

With the exception of germ cells, any somatic cell can be used as a starting point for iPSCs. For example, cell types could be keratinocytes, fibroblasts, hematopoietic cells, mesenchymal cells, liver cells, or stomach cells. T cells may also be used as a source of somatic cells for reprogramming (U.S. Patent No. 8,741,648). There is no limitation on the degree of cell differentiation or the age of an animal from which cells are collected; even undifferentiated progenitor cells (including somatic stem cells) and finally differentiated mature cells can be used as sources of somatic cells in the methods disclosed herein. In one embodiment, the somatic cell is itself a RPE or PRP cell, such as a human PRP cell. The RPE or PRP cell can be an adult or a fetal RPE or PRP cell. iPSCs can be grown under conditions that are known to differentiate human ES cells into specific cell types, and express human ES cell markers including: SSEA-1, SSEA-3, SSEA-4, TRA-1-60, and TRA-1-81.

### A. HLA of Starting Cells

Major Histocompatibility Complex (MHC) is the main cause of immune-rejection of allogeneic organ transplants. There are three major class I MHC haplotypes (A, B, and C) and three major MHC class II haplotypes (DR, DP, and DQ).

MHC compatibility between a donor and a recipient increases significantly if the donor cells are HLA homozygous, *i.e.* contain identical alleles for each antigen-presenting protein. Most individuals are heterozygous for MHC class I and II genes, but certain individuals are homozygous for these genes. These homozygous individuals can serve as super donors, and grafts generated from their cells can be transplanted in all individuals that are either homozygous or heterozygous for that haplotype. Furthermore, if homozygous donor cells have a haplotype found in high frequency in a population, these cells may have application in transplantation therapies for a large number of individuals.

Accordingly, the iPSCs can be produced from somatic cells of the subject to be treated, or another subject with the same or substantially the same HLA type as that of the patient. In one case, the major HLAs (e.g., the three major loci of HLA-A, HLA-B and HLA-DR) of the donor are identical to the major HLAs of the recipient. In some cases, the somatic cell donor may be a super donor; thus, iPSCs derived from a MHC homozygous super donor may be used to generate RPE or PRP cells. Thus, the iPSCs derived from a super donor may be transplanted in subjects that are either homozygous or heterozygous for that haplotype. For example, the iPSCs can be homozygous at two HLA alleles such as HLA-A and HLA-B. As such, iPSCs produced from super donors can be used in the methods disclosed herein, to produce RPE or PRP cells that can potentially "match" a large number of potential recipients.

### B. Reprogramming Factors

Somatic cells can be reprogrammed to produce induced pluripotent stem cells (iPSCs) using methods known to one of skill in the art. One of skill in the art can readily produce induced pluripotent stem cells; see for example, Published U.S. Patent Application No. 20090246875, Published U.S. Patent Application No. 2010/0210014; Published U.S. Patent Application No. 20120276636; U.S. Patent No. 8,058,065; U.S. Patent No. 8,129,187; U.S. Patent No. 8,278,620; PCT Publication NO. WO 2007/069666 A1, and U.S. Patent No. 8,268,620. Generally, nuclear reprogramming factors are used to produce pluripotent stem cells from a somatic cell. In some embodiments, at least two, at least three, or at least four, of Klf4, c-Myc, Oct3/4, Sox2, Nanog, and Lin28 are utilized. In other embodiments, Oct3/4, Sox2, c-Myc and Klf4 are utilized.

The cells are treated with a nuclear reprogramming substance, which is generally one or more factor(s) capable of inducing an iPSC from a somatic cell or a nucleic acid that encodes these substances (including forms integrated in a vector). The nuclear reprogramming substances generally include at least Oct3/4, Klf4 and Sox2 or nucleic acids that encode these molecules. A functional inhibitor of p53, L-myc or a nucleic acid that encodes L-myc, and Lin28 or Lin28b or a nucleic acid that encodes Lin28 or Lin28b, can be utilized as additional nuclear reprogramming substances. Nanog can also be utilized for nuclear reprogramming. As disclosed in published U.S. Patent Application No. 20120196360, exemplary reprogramming factors for the production of iPSCs include (1) Oct3/4, Klf4, Sox2, L-Myc (Sox2 can be replaced with Sox1, Sox3, Soxl5, Soxl7 or Soxl8; Klf4 is replaceable with K1f1, Klf2 or Klf5); (2) Oct3/4, Klf4, Sox2, L-Myc, TERT, SV40 Large T antigen (SV40LT); (3) Oct3/4, Klf4, Sox2, L-Myc, TERT, human papilloma virus (HPV)16 E6; (4) Oct3/4, Klf4, Sox2, L-Myc, TERT, HPV16 E7 (5) Oct3/4, Klf4, Sox2, L- Myc, TERT, HPV16 E6, HPV16 E7; (6) Oct3/4, Klf4, Sox2, L-Myc, TERT, Bmil; (7) Oct3/4, Klf4, Sox2, L-Myc, Lin28; (8) Oct3/4, Klf4, Sox2, L-Myc, Lin28, SV40LT; (9) Oct3/4, Klf4, Sox2, L-Myc, Lin28, TERT, SV40LT; (10) Oct3/4, Klf4, Sox2, L-Myc, SV40LT; (11) Oct3/4, Esrrb, Sox2, L-Myc (Esrrb is replaceable with Esrrg); (12) Oct3/4, Klf4, Sox2; (13) Oct3/4, Klf4, Sox2, TERT, SV40LT; (14) Oct3/4, Klf4, Sox2, TERT, HP VI 6 E6; (15) Oct3/4, Klf4, Sox2, TERT, HPV16 E7; (16) Oct3/4, Klf4, Sox2, TERT, HPV16 E6, HPV16 E7; (17) Oct3/4, Klf4, Sox2, TERT, Bmil; (18) Oct3/4, Klf4, Sox2, Lin28 (19) Oct3/4, Klf4, Sox2, Lin28, SV40LT; (20) Oct3/4, Klf4, Sox2, Lin28, TERT, SV40LT; (21) Oct3/4, Klf4, Sox2, SV40LT; or (22) Oct3/4, Esrrb, Sox2 (Esrrb is replaceable with Esrrg). In one non-limiting example, Oct3/4, Klf4, Sox2, and c-Myc are utilized. In other embodiments, Oct4, Nanog, and Sox2 are utilized; see for example, U.S. Patent No. 7,682,828. These factors include, but are not limited to, Oct3/4, Klf4 and Sox2. In other examples, the factors include, but are not limited to Oct 3/4, Klf4 and Myc. In some non-limiting examples, Oct3/4, Klf4, c-Myc, and Sox2 are utilized. In other non-limiting examples, Oct3/4, Klf4, Sox2 and Sal 4 are utilized. Factors like Nanog, Lin28, Klf4, or c-Myc can increase reprogramming efficiency and can be expressed from several different expression vectors. For example, an integrating vector such as the EBV element-based system can be used (U.S. Patent No. 8,546,140). In a further aspect, reprogramming proteins could be introduced directly into somatic cells by protein transduction. Reprogramming may further comprise contacting the cells with one or more signaling receptors including glycogen synthase kinase 3 (GSK-3) inhibitor, a mitogen-activated protein kinase kinase (MEK) inhibitor, a transforming growth factor beta (TGF-β) receptor inhibitor or signaling inhibitor, leukemia inhibitory factor (LIF), a p53 inhibitor, an NF-kappa B inhibitor, or a combination thereof. Those regulators may include small molecules, inhibitory nucleotides, expression cassettes, or protein factors. It is anticipated that virtually any iPS cells or cell lines may be used.

Mouse and human cDNA sequences of these nuclear reprogramming substances are available with reference to the NCBI accession numbers mentioned in WO 2007/069666. Methods for introducing one or more reprogramming substances, or nucleic acids encoding these reprogramming substances, are known in the art, and disclosed for example, in published U.S. Patent Application No. 2012/0196360 and U.S. Patent No. 8,071,369.

Once derived, iPSCs can be cultured in a medium sufficient to maintain pluripotency. The iPSCs may be used with various media and techniques developed to culture pluripotent stem cells, more specifically, embryonic stem cells, as described in U.S. Patent No. 7,442,548 and U.S. Patent Pub. No. 2003/0211603. In the case of mouse cells, the culture is carried out with the addition of Leukemia Inhibitory Factor (LIF) as a differentiation suppression factor to an ordinary medium. In the case of human cells, it is desirable that basic fibroblast growth factor (bFGF) be added in place of LIF. Other methods for the culture and maintenance of iPSCs, as would be known to one of skill in the art, may be used.

In certain embodiments, undefined conditions may be used; for example, pluripotent cells may be cultured on fibroblast feeder cells or a medium that has been exposed to fibroblast feeder cells in order to maintain the stem cells in an undifferentiated state. In some embodiments, the cell is cultured in the co-presence of mouse embryonic fibroblasts treated with radiation or an antibiotic to terminate the cell division, as feeder cells. Alternately, pluripotent cells may be cultured and maintained in an essentially undifferentiated state using a defined, feeder-independent culture system, such as a TESR^{™} medium (Ludwig *et al*., 2006a; Ludwig *et al*., 2006b) or E8^{™} medium (Chen *et al*., 2011).

### C. Plasmids

In some embodiments, the iPSC can be modified to express exogenous nucleic acids, such as to include an enhancer operably linked to a promoter and a nucleic acid sequence encoding a first marker. Suitable promoters include, but are not limited to, any promoter expressed in photoreceptor cells, such as a rhodopsin kinase promoter. The construct can also include other elements, such as a ribosome binding site for translational initiation (internal ribosomal binding sequences), and a transcription/translation terminator. Generally, it is advantageous to transfect cells with the construct. Suitable vectors for stable transfection include, but are not limited to retroviral vectors, lentiviral vectors and Sendai virus.

In some embodiments plasmids that encode a marker are composed of: (1) a high copy number replication origin, (2) a selectable marker, such as, but not limited to, the neo gene for antibiotic selection with kanamycin, (3) transcription termination sequences, including the tyrosinase enhancer and (4) a multicloning site for incorporation of various nucleic acid cassettes; and (5) a nucleic acid sequence encoding a marker operably linked to the tyrosinase promoter. There are numerous plasmid vectors that are known in the art for inducing a nucleic acid encoding a protein. These include, but are not limited to, the vectors disclosed in U.S. Patent No. 6,103,470; U.S. Patent No. 7,598,364; U.S. Patent No. 7,989,425; and U.S. Patent No. 6,416,998.

A viral gene delivery system can be an RNA-based or DNA-based viral vector. An episomal gene delivery system can be a plasmid, an Epstein-Barr virus (EBV)-based episomal vector, a yeast-based vector, an adenovirus-based vector, a simian virus 40 (SV40)-based episomal vector, a bovine papilloma virus (BPV)-based vector, or a lentiviral vector.

Markers include, but are not limited to, fluorescence proteins (for example, green fluorescent protein or red fluorescent protein), enzymes (for example, horse radish peroxidase or alkaline phosphatase or firefly/renilla luciferase or nanoluc), or other proteins. A marker may be a protein (including secreted, cell surface, or internal proteins; either synthesized or taken up by the cell); a nucleic acid (such as an mRNA, or enzymatically active nucleic acid molecule) or a polysaccharide. Included are determinants of any such cell components that are detectable by antibody, lectin, probe or nucleic acid amplification reaction that are specific for the marker of the cell type of interest. The markers can also be identified by a biochemical or enzyme assay or biological response that depends on the function of the gene product. Nucleic acid sequences encoding these markers can be operably linked to the tyrosinase enhancer. In addition, other genes can be included, such as genes that may influence stem cell to PRP differentiation, or photoreceptor function, or physiology, or pathology.

### D. Delivery Systems

Introduction of a nucleic acid, such as DNA or RNA, into the pluripotent stem cells to be programmed to RPE or PRPs with the current disclosure may use any suitable methods for nucleic acid delivery for transformation of a cell, as described herein or as would be known to one of ordinary skill in the art. Such methods include, but are not limited to, direct delivery of DNA such as by *ex vivo* transfection (Wilson *et al*., 1989, Nabel *et al*, 1989), by injection (U.S. Patent Nos. 5,994,624, 5,981,274, 5,945,100, 5,780,448, 5,736,524, 5,702,932, 5,656,610, 5,589,466 and 5,580,859), including microinjection (Harland and Weintraub, 1985; U.S. Patent No. 5,789,215); by electroporation (U.S. Patent No. 5,384,253; Tur-Kaspa *et al*., 1986; Potter *et al*., 1984); by calcium phosphate precipitation (Graham and Van Der Eb, 1973; Chen and Okayama, 1987; Rippe *et al*., 1990); by using DEAE-dextran followed by polyethylene glycol (Gopal, 1985); by direct sonic loading (Fechheimer *et al*., 1987); by liposome mediated transfection (Nicolau and Sene, 1982; Fraley *et al*., 1979; Nicolau *et al*., 1987; Wong *et al*., 1980; Kaneda *et al*., 1989; Kato *et al*., 1991) and receptor-mediated transfection (Wu and Wu, 1987; Wu and Wu, 1988); by microprojectile bombardment (PCT Application Nos. WO 94/09699 and 95/06128; U.S. Patent Nos. 5,610,042; 5,322,783 5,563,055, 5,550,318, 5,538,877 and 5,538,880); by agitation with silicon carbide fibers (Kaeppler et al., 1990; U.S. Patent Nos. 5,302,523 and 5,464,765); by *Agrobacterium-mediated* transformation (U.S. Patent Nos. 5,591,616 and 5,563,055); by desiccation/inhibition-mediated DNA uptake (Potrykus *et al*., 1985), and any combination of such methods. Through the application of techniques such as these, organelle(s), cell(s), tissue(s) or organism(s) may be stably or transiently transformed.

### 1. Viral Vectors

Viral vectors may be provided in certain aspects of the present disclosure. In generating recombinant viral vectors, non-essential genes are typically replaced with a gene or coding sequence for a heterologous (or non-native) protein. A viral vector is a kind of expression construct that utilizes viral sequences to introduce nucleic acid and possibly proteins into a cell. The ability of certain viruses to infect cells or enter cells via receptor-mediated endocytosis, and to integrate into host cell genomes and express viral genes stably and efficiently have made them attractive candidates for the transfer of foreign nucleic acids into cells (e.g., mammalian cells). Non-limiting examples of virus vectors that may be used to deliver a nucleic acid of certain aspects of the present disclosure are described below.

Retroviruses have promise as gene delivery vectors due to their ability to integrate their genes into the host genome, transfer a large amount of foreign genetic material, infect a broad spectrum of species and cell types, and be packaged in special cell-lines (Miller, 1992).

In order to construct a retroviral vector, a nucleic acid is inserted into the viral genome in place of certain viral sequences to produce a virus that is replication-defective. In order to produce virions, a packaging cell line containing the gag, pol, and env genes-but without the LTR and packaging components-is constructed (Mann *et al*., 1983). When a recombinant plasmid containing a cDNA, together with the retroviral LTR and packaging sequences, is introduced into a special cell line *(e.g.,* by calcium phosphate precipitation), the packaging sequence allows the RNA transcript of the recombinant plasmid to be packaged into viral particles, which are then secreted into the culture medium (Nicolas and Rubenstein, 1988; Temin, 1986; Mann *et al*., 1983). The medium containing the recombinant retroviruses is then collected, optionally concentrated, and used for gene transfer. Retroviral vectors are able to infect a broad variety of cell types. However, integration and stable expression require the division of host cells (Paskind *et al*., 1975).

Lentiviruses are complex retroviruses, which, in addition to the common retroviral genes *gag, pol,* and *env,* contain other genes with regulatory or structural function. Lentiviral vectors are well known in the art (see, for example, Naldini *et al*., 1996; Zufferey *et al*., 1997; Blomer *et al*., 1997; U.S. Patents 6,013,516 and 5,994,136).

Recombinant lentiviral vectors are capable of infecting non-dividing cells and can be used for both *in vivo* and *ex vivo* gene transfer and expression of nucleic acid sequences. For example, recombinant lentivirus capable of infecting a non-dividing cell-wherein a suitable host cell is transfected with two or more vectors carrying the packaging functions, namely gag, pol and env, as well as rev and tat-is described in U.S. Patent 5,994,136.

### 2. Episomal Vectors

The use of plasmid- or liposome-based extra-chromosomal (*i.e*., episomal) vectors may be also provided in certain aspects of the present disclosure. Such episomal vectors may include, *e.g.,* oriP-based vectors, and/or vectors encoding a derivative of EBNA-1. These vectors may permit large fragments of DNA to be introduced unto a cell and maintained extra-chromosomally, replicated once per cell cycle, partitioned to daughter cells efficiently, and elicit substantially no immune response.

In particular, EBNA-1, the only viral protein required for the replication of the oriP-based expression vector, does not elicit a cellular immune response because it has developed an efficient mechanism to bypass the processing required for presentation of its antigens on MHC class I molecules (Levitskaya *et al*., 1997). Further, EBNA-1 can act in *trans* to enhance expression of the cloned gene, inducing expression of a cloned gene up to 100-fold in some cell lines (Langle-Rouault *et al*., 1998; Evans *et al*., 1997). Finally, the manufacture of such oriP-based expression vectors is inexpensive.

In certain aspects, reprogramming factors are expressed from expression cassettes comprised in one or more exogenous episiomal genetic elements (see U.S. Patent Publication 2010/0003757,). Thus, iPSCs can be essentially free of exogenous genetic elements, such as from retroviral or lentiviral vector elements. These iPSCs are prepared by the use of extra-chromosomally replicating vectors (*i.e.,* episomal vectors), which are vectors capable of replicating episomally to make iPSCs essentially free of exogenous vector or viral elements (see U.S. Patent No. 8,546,140, Yu *et al*., 2009). A number of DNA viruses, such as adenoviruses, Simian vacuolating virus 40 (SV40) or bovine papilloma virus (BPV), or budding yeast ARS (Autonomously Replicating Sequences)-containing plasmids replicate extra-chromosomally or episomally in mammalian cells. These episomal plasmids are intrinsically free from all these disadvantages (Bode *et al*., 2001) associated with integrating vectors. For example, a lymphotrophic herpes virus-based including or Epstein Barr Virus (EBV) as defined above may replicate extra-chromosomally and help deliver reprogramming genes to somatic cells. Useful EBV elements are OriP and EBNA-1, or their variants or functional equivalents. An additional advantage of episomal vectors is that the exogenous elements will be lost with time after being introduced into cells, leading to self-sustained iPSCs essentially free of these elements.

Other extra-chromosomal vectors include other lymphotrophic herpes virus-based vectors. Lymphotrophic herpes virus is a herpes virus that replicates in a lymphoblast (*e.g.,* a human B lymphoblast) and becomes a plasmid for a part of its natural life-cycle. Herpes simplex virus (HSV) is not a "lymphotrophic" herpes virus. Exemplary lymphotrophic herpes viruses include, but are not limited to EBV, Kaposi's sarcoma herpes virus (KSHV); Herpes virus saimiri (HS) and Marek's disease virus (MDV). Also, other sources of episome-based vectors are contemplated, such as yeast ARS, adenovirus, SV40, or BPV.

One of skill in the art would be well-equipped to construct a vector through standard recombinant techniques (*see,* for example, Maniatis *et al*., 1988 and Ausubel *et al*., 1994).

Vectors can also comprise other components or functionalities that further modulate gene delivery and/or gene expression, or that otherwise provide beneficial properties to the targeted cells. Such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide.

Such components also may include markers, such as detectable and/or selection markers that can be used to detect or select for cells that have taken up and are expressing the nucleic acid delivered by the vector. Such components can be provided as a natural feature of the vector (such as the use of certain viral vectors that have components or functionalities mediating binding and uptake), or vectors can be modified to provide such functionalities. A large variety of such vectors are known in the art and are generally available. When a vector is maintained in a host cell, the vector can either be stably replicated by the cells during mitosis as an autonomous structure, incorporated within the genome of the host cell, or maintained in the host cell's nucleus or cytoplasm.

### 3. Regulatory Elements

Expression cassettes included in reprogramming vectors useful in the present disclosure preferably contain (in a 5'-to-3' direction) a eukaryotic transcriptional promoter operably linked to a protein-coding sequence, splice signals including intervening sequences, and a transcriptional termination/polyadenylation sequence.

### b. Promoter/Enhancers

The expression constructs provided herein comprise promoter to drive expression of the programming genes. A promoter generally comprises a sequence that functions to position the start site for RNA synthesis. The best known example of this is the TATA box, but in some promoters lacking a TATA box, such as, for example, the promoter for the mammalian terminal deoxynucleotidyl transferase gene and the promoter for the SV40 late genes, a discrete element overlying the start site itself helps to fix the place of initiation. Additional promoter elements regulate the frequency of transcriptional initiation. Typically, these are located in the region 30-110 bp upstream of the start site, although a number of promoters have been shown to contain functional elements downstream of the start site as well. To bring a coding sequence "under the control of" a promoter, one positions the 5' end of the transcription initiation site of the transcriptional reading frame "downstream" of (*i.e.,* 3' of) the chosen promoter. The "upstream" promoter stimulates transcription of the DNA and promotes expression of the encoded RNA.

The spacing between promoter elements frequently is flexible, so that promoter function is preserved when elements are inverted or moved relative to one another. In the tk promoter, the spacing between promoter elements can be increased to 50 bp apart before activity begins to decline. Depending on the promoter, it appears that individual elements can function either cooperatively or independently to activate transcription. A promoter may or may not be used in conjunction with an "enhancer," which refers to a cis-acting regulatory sequence involved in the transcriptional activation of a nucleic acid sequence.

A promoter may be one naturally associated with a nucleic acid sequence, as may be obtained by isolating the 5' non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other virus, or prokaryotic or eukaryotic cell, and promoters or enhancers not "naturally occurring," *i.e.,* containing different elements of different transcriptional regulatory regions, and/or mutations that alter expression. For example, promoters that are most commonly used in recombinant DNA construction include the β-lactamase (penicillinase), lactose and tryptophan (trp) promoter systems. In addition to producing nucleic acid sequences of promoters and enhancers synthetically, sequences may be produced using recombinant cloning and/or nucleic acid amplification technology, including PCR^{™}, in connection with the compositions disclosed herein (*see* U.S. Patent Nos. 4,683,202 and 5,928,906). Furthermore, it is contemplated that the control sequences that direct transcription and/or expression of sequences within non-nuclear organelles such as mitochondria, chloroplasts, and the like, can be employed as well.

Naturally, it will be important to employ a promoter and/or enhancer that effectively directs the expression of the DNA segment in the organelle, cell type, tissue, organ, or organism chosen for expression. Those of skill in the art of molecular biology generally know the use of promoters, enhancers, and cell type combinations for protein expression, (*see,* for example Sambrook *et al.* 1989). The promoters employed may be constitutive, tissue-specific, inducible, and/or useful under the appropriate conditions to direct high level expression of the introduced DNA segment, such as is advantageous in the large-scale production of recombinant proteins and/or peptides. The promoter may be heterologous or endogenous.

Additionally any promoter/enhancer combination (as per, for example, the Eukaryotic Promoter Data Base EPDB) could also be used to drive expression. Use of a T3, T7 or SP6 cytoplasmic expression system is another possible embodiment. Eukaryotic cells can support cytoplasmic transcription from certain bacterial promoters if the appropriate bacterial polymerase is provided, either as part of the delivery complex or as an additional genetic expression construct.

Non-limiting examples of promoters include early or late viral promoters, such as, SV40 early or late promoters, cytomegalovirus (CMV) immediate early promoters, Rous Sarcoma Virus (RSV) early promoters; eukaryotic cell promoters, such *as, e.* g., beta actin promoter (Ng, 1989; Quitsche *et al*., 1989), GADPH promoter (Alexander *et al*., 1988, Ercolani *et al*., 1988), metallothionein promoter (Karin *et al*., 1989; Richards *et al*., 1984); and concatenated response element promoters, such as cyclic AMP response element promoters (cre), serum response element promoter (sre), phorbol ester promoter (TPA) and response element promoters (tre) near a minimal TATA box. It is also possible to use human growth hormone promoter sequences (e.g., the human growth hormone minimal promoter described at Genbank, accession no. X05244, nucleotide 283-341) or a mouse mammary tumor promoter (available from the ATCC, Cat. No. ATCC 45007).

Tissue-specific transgene expression, especially for reporter gene expression in hematopoietic cells and precursors of hematopoietic cells derived from programming, may be desirable as a way to identify derived hematopoietic cells and precursors. To increase both specificity and activity, the use of cis-acting regulatory elements has been contemplated. For example, a hematopoietic cell-specific promoter may be used. Many such hematopoietic cell-specific promoters are known in the art.

In certain aspects, methods of the present disclosure also concern enhancer sequences, *i.e.,* nucleic acid sequences that increase a promoter's activity and that have the potential to act in cis, and regardless of their orientation, even over relatively long distances (up to several kilobases away from the target promoter). However, enhancer function is not necessarily restricted to such long distances as they may also function in close proximity to a given promoter.

Many hematopoietic cell promoter and enhancer sequences have been identified, and may be useful in present methods. *See, e.g.,* U.S. Patent 5,556,954; U.S. Patent App. 20020055144; U.S. Patent App. 20090148425.

### c. Initiation Signals and Linked Expression

A specific initiation signal also may be used in the expression constructs provided in the present disclosure for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals. It is well known that the initiation codon must be "in-frame" with the reading frame of the desired coding sequence to ensure translation of the entire insert. The exogenous translational control signals and initiation codons can be either natural or synthetic. The efficiency of expression may be enhanced by the inclusion of appropriate transcription enhancer elements.

In certain embodiments, internal ribosome entry sites (IRES) elements are used to create multigene, or polycistronic, messages. IRES elements are able to bypass the ribosome scanning model of 5' methylated Cap dependent translation and begin translation at internal sites (Pelletier and Sonenberg, 1988). IRES elements from two members of the picornavirus family (polio and encephalomyocarditis) have been described (Pelletier and Sonenberg, 1988), as well an IRES from a mammalian message (Macejak and Sarnow, 1991). IRES elements can be linked to heterologous open reading frames. Multiple open reading frames can be transcribed together, each separated by an IRES, creating polycistronic messages. By virtue of the IRES element, each open reading frame is accessible to ribosomes for efficient translation. Multiple genes can be efficiently expressed using a single promoter/enhancer to transcribe a single message (see U.S. Patent Nos. 5,925,565 and 5,935,819).

Additionally, certain 2A sequence elements could be used to create linked- or co-expression of programming genes in the constructs provided in the present disclosure. For example, cleavage sequences could be used to co-express genes by linking open reading frames to form a single cistron. An exemplary cleavage sequence is the F2A (Foot- and-mouth diease virus 2A) or a "2A-like" sequence (*e.g., Thosea asigna* virus 2A; T2A) (Minskaia and Ryan, 2013). In particular embodiments, an F2A-cleavage peptide is used to link expression of the genes in the multi-lineage construct.

### d. Origins of Replication

In order to propagate a vector in a host cell, it may contain one or more origins of replication sites (often termed "ori"), for example, a nucleic acid sequence corresponding to oriP of EBV as described above or a genetically engineered oriP with a similar or elevated function in programming, which is a specific nucleic acid sequence at which replication is initiated. Alternatively a replication origin of other extra-chromosomally replicating virus as described above or an autonomously replicating sequence (ARS) can be employed.

### e. Selection and Screenable Markers

In certain embodiments, cells containing a nucleic acid construct may be identified *in vitro* or *in vivo* by including a marker in the expression vector. Such markers would confer an identifiable change to the cell permitting easy identification of cells containing the expression vector. Generally, a selection marker is one that confers a property that allows for selection. A positive selection marker is one in which the presence of the marker allows for its selection, while a negative selection marker is one in which its presence prevents its selection. An example of a positive selection marker is a drug resistance marker.

Usually the inclusion of a drug selection marker aids in the cloning and identification of transformants, for example, genes that confer resistance to neomycin, puromycin, hygromycin, DHFR, GPT, zeocin and histidinol are useful selection markers. In addition to markers conferring a phenotype that allows for the discrimination of transformants based on the implementation of conditions, other types of markers including screenable markers such as GFP, whose basis is colorimetric analysis, are also contemplated. Alternatively, screenable enzymes as negative selection markers such as herpes simplex virus thymidine kinase (*tk*) or chloramphenicol acetyltransferase (CAT) may be utilized. One of skill in the art would also know how to employ immunologic markers, possibly in conjunction with FACS analysis. The marker used is not believed to be important, so long as it is capable of being expressed simultaneously with the nucleic acid encoding a gene product. Further examples of selection and screenable markers are well known to one of skill in the art.

### III. Differentiation of iPSCs to Retinal Pigment Epithelial Cells, Photoreceptors or Photoreceptor Precursor Cells

### A. RPE Differentiation

In some aspects, RPE cells are produced in the methods disclosed herein from iPSCs. The cells in the retina that are directly sensitive to light are the photoreceptor cells. Photoreceptors are photosensitive neurons in the outer part of the retina and can be either rods or cones. In the process of phototransduction, the photoreceptor cells convert incident light energy focused by the cornea and lens to electric signals which are ultimately sent via the optic nerve to the brain. Vertebrates have two types of photoreceptor cells including cones and rods. Cones are adapted to detect fine detail, central and color vision and function well in bright light. Rods are responsible for peripheral and dim light vision. Neural signals from the rods and cones undergo processing by other neurons of the retina.

The retinal pigment epithelium acts as a barrier between the bloodstream and the retina and closely interacts with photoreceptors in the maintenance of visual function. The retinal pigment epithelium is composed of a single layer of hexagonal cells that are densely packed with granules of melanin that absorbs light energy that arrives to the retina. The main functions of the specialized RPE cells include: transport of nutrients such as glucose, retinol, and fatty acids from the blood to the photoreceptors; transport of water, metabolic end products, and ions from the subretinal space to the blood; absorption of light and protection against photooxidation; reisomerization of all-trans-retinol into 11-cis-retinal; phagocytosis of shed photoreceptor membranes; and secretion of various essential factors for the structural integrity of the retina.

The retinal pigment epithelium expresses markers such as cellular retinaldehyde-binding protein (CRALBP), RPE65, best vitelliform macular dystrophy gene (VMD2), and pigment epithelium derived factor (PEDF). Malfunction of the retinal pigment epithelium is associated with a number of vision-altering conditions, such as retinal pigment epithelium detachment, dysplasia, atrophy, retinopathy, retinitis pigmentosa, macular dystrophy, or degeneration, including age-related macular degeneration.

Retinal pigment epithelial (RPE) cells can be characterized based upon their pigmentation, epithelial morphology, and apical-basal polarity. Differentiated RPE cells can be visually recognized by their cobblestone morphology and the initial appearance of pigment. In addition, differentiated RPE cells have transepithelial resistance/TER, and transepithelial potential/TEP across the monolayer (TER > 100 ohms. cm2; TEP >2 mV), transport fluid and CO₂ from the apical to basal side, and regulate a polarized secretion of cytokines.

RPE cells express several proteins that can serve as markers for detection by the use of methodologies such as immunocytochemistry, Western blot analysis, flow cytometry, and enzyme-linked immunoassay (ELISA). For example, RPE-specific markers may include: cellular retinaldehyde binding protein (CRALBP), microphthalmia-associated transcription factor (MITF), tyrosinase-related protein 1 (TYRP-1), retinal pigment epithelium-specific 65 kDa protein (RPE65), premelanosome protein (PMEL17), bestrophin 1 (BEST1), and c-mer proto-oncogene tyrosine kinase (MERTK). RPE cells do not express (at any detectable level) the embryonic stem cells markers Oct-4, nanog or Rex-1. Specifically, expression of these genes is approximately 100-1000 fold lower in RPE cells than in ES cells or iPSC cells, when assessed by quantitative RT-PCR.

RPE cell markers may be detected at the mRNA level, for example, by reverse transcriptase polymerase chain reaction (RT-PCR), Northern blot analysis, or dot-blot hybridization analysis using sequence-specific primers in standard amplification methods using publicly available sequence data (GENBANK^{®}). Expression of tissue-specific markers as detected at the protein or mRNA level is considered positive if the level is at least or about 2-, 3-, 4-, 5-, 6-, 7-, 8-, or 9-fold, and more particularly more than 10-, 20-, 30, 40-, 50-fold or higher above that of a control cell, such as an undifferentiated pluripotent stem cell or other unrelated cell type.

Dysfunction, injury, and loss of RPE cells are factors of many eye diseases and disorders including age-related macular degeneration (AMD), hereditary macular degenerations including Best disease, Stargardt disease and choroideremia, and other forms of inherited retinal diseases and acquired retinal dysfunctions, diseases, and injuries, including but not limited to RPE tears/rips. A potential treatment for such diseases is the transplantation of RPE cells into the retina of those in need of such treatment. It is speculated that the replenishment of RPE cells by their transplantation may delay, halt or reverse degradation, improve retinal function and prevent blindness stemming from such conditions. However, obtaining RPE cells directly from human donors and embryos is a challenge.

### 2. Derivation of RPE cells from Embryoid Bodies of PSCs

iPSCs reprogrammed using well-known reprogramming factors can give rise to ocular cells of neuronal lineage, including RPE cells (Hirami *et al*., 2009). PCT Publication No. 2014/121077 discloses methods wherein embryoid bodies (EBs) produced from iPSCs are treated with Wnt and Nodal antagonists in suspension culture to induce expression of markers of retinal progenitor cells. This publication discloses methods wherein RPE cells are derived from iPSCs through a process of differentiation of EBs of the iPSCs into cultures highly enriched for RPE cells. For example, embryoid bodies are produced from iPSCs by the addition of a rho-associated coiled-coil kinase (ROCK) inhibitor and cultured in a first medium comprising two WNT pathway inhibitors and a Nodal pathway inhibitor. Further, the EBs are plated on a MATRIGEL^{™} coated tissue culture in a second medium that does not comprise basic fibroblast growth factor (bFGF), comprises a Nodal pathway inhibitor, comprises about 20 ng to about 90 ng of Noggin, and comprises about 1 to about 5% knock out serum replacement to form differentiating RPE cells. The differentiating RPE cells are cultured in a third medium comprising ACTIVIN and WNT3a. The RPE cells are then cultured in RPE medium that includes about 5% fetal serum, a canonical WNT inhibitor, a non-canonical WNT inhibitor, and inhibitors of the Sonic Hedgehog and FGF pathways to produce human RPE cells.

There are several disadvantages in the use of EBs for the production of differentiated cell type. For example, the production of EBs from iPSCs is a non-consistent and non-reproducible process as the efficiency varies and size or shape of EBs vary. The present disclosure provides methods that allow large-scale production of iPSC- or ES-derived cells needed for clinical, research or therapeutic applications that are independent of EBs.

### 3. Derivation of RPE cells from Essentially Single Cell PSCs

In some embodiments, methods are provided for producing RPE cells from an essentially single cell suspension of pluripotent stem cells (PSCs) such as human iPSCs. In some embodiments, the PSCs are cultured to pre-confluence to prevent any cell aggregates. In certain aspects, the PSCs are dissociated by incubation with a cell dissociation enzyme, such as exemplified by TRYPSIN or TRYPLE^{™}. PSCs can also be dissociated into an essentially single cell suspension by pipetting. In addition, Blebbistatin (*e.g*., about 2.5 µM) can be added to the medium to increase PSC survival after dissociation into single cells while the cells are not adhered to a culture vessel. A ROCK inhibitor instead of Blebbistatin may alternatively be used to increase PSC survival after dissociation into single cells.

Once a single cell suspension of PSCs is obtained, the cells are generally seeded in an appropriate culture vessel, such as a tissue culture plate, such as a flask, 6-well, 24-well, or 96-well plate. A culture vessel used for culturing the cell(s) can include, but is particularly not limited to: flask, flask for tissue culture, dish, petri dish, dish for tissue culture, multi dish, micro plate, micro-well plate, multi plate, multi-well plate, micro slide, chamber slide, tube, tray, CELLSTACK^{®} Chambers, culture bag, and roller bottle, as long as it is capable of culturing the stem cells therein. The cells may be cultured in a volume of at least or about 0.2, 0.5, 1, 2, 5, 10, 20, 30, 40, 50 ml, 100 ml, 150 ml, 200 ml, 250 ml, 300 ml, 350 ml, 400 ml, 450 ml, 500 ml, 550 ml, 600 ml, 800 ml, 1000 ml, 1500 ml, or any range derivable therein, depending on the needs of the culture. In a certain embodiment, the culture vessel may be a bioreactor, which may refer to any device or system *ex vivo* that supports a biologically active environment such that cells can be propagated. The bioreactor may have a volume of at least or about 2, 4, 5, 6, 8, 10, 15, 20, 25, 50, 75, 100, 150, 200, 500 liters, 1, 2, 4, 6, 8, 10, 15 cubic meters, or any range derivable therein.

In certain aspects, the PSCs, such as iPSCs, are plated at a cell density appropriate for efficient differentiation. Generally, the cells are plated at a cell density of about 1,000 to about 75,000 cells/cm², such as of about 5,000 to about 40,000 cells/cm². In a 6 well plate, the cells may be seeded at a cell density of about 50,000 to about 400,000 cells per well. In exemplary methods, the cells are seeded at a cell density of about 100,000, about 150,000, about 200,000, about 250,000, about 300,000 or about 350,000 cells per well, such as about 200,000 cells per well.

The PSCs, such as iPSCs, are generally cultured on culture plates coated by one or more cellular adhesion proteins to promote cellular adhesion while maintaining cell viability. For example, preferred cellular adhesion proteins include extracellular matrix proteins such as vitronectin, laminin, collagen and/or fibronectin which may be used to coat a culturing surface as a means of providing a solid support for pluripotent cell growth. The term "extracellular matrix" is recognized in the art. Its components include one or more of the following proteins: fibronectin, laminin, vitronectin, tenascin, entactin, thrombospondin, elastin, gelatin, collagen, fibrillin, merosin, anchorin, chondronectin, link protein, bone sialoprotein, osteocalcin, osteopontin, epinectin, hyaluronectin, undulin, epiligrin, and kalinin. In exemplary methods, the PSCs are grown on culture plates coated with vitronectin or fibronectin. In some embodiments, the cellular adhesion proteins are human proteins.

The extracellular matrix (ECM) proteins may be of natural origin and purified from human or animal tissues or, alternatively, the ECM proteins may be genetically engineered recombinant proteins or synthetic in nature. The ECM proteins may be a whole protein or in the form of peptide fragments, native or engineered. Examples of ECM protein that may be useful in the matrix for cell culture include laminin, collagen I, collagen IV, fibronectin and vitronectin. In some embodiments, the matrix composition includes synthetically generated peptide fragments of fibronectin or recombinant fibronectin. In some embodiments, the matrix composition is xeno-free. For example, in the xeno-free matrix to culture human cells, matrix components of human origin may be used, wherein any non-human animal components may be excluded.

In some aspects, the total protein concentration in the matrix composition may be about 1 ng/mL to about 1 mg/mL. In some preferred embodiments, the total protein concentration in the matrix composition is about 1 µg/mL to about 300 µg/mL. In more preferred embodiments, the total protein concentration in the matrix composition is about 5 µg/mL to about 200 µg/mL.

Cells, such as RPE cells or PSC, can be cultured with the nutrients necessary to support the growth of each specific population of cells. Generally, the cells are cultured in growth media including a carbon source, a nitrogen source and a buffer to maintain pH. The medium can also contain fatty acids or lipids, amino acids (such as non-essential amino acids), vitamin(s), growth factors, cytokines, antioxidant substances, pyruvic acid, buffering agents, and inorganic salts. An exemplary growth medium contains a minimal essential media, such as Dulbecco's Modified Eagle's medium (DMEM) or ESSENTIAL 8^{™} (E8^{™}) medium, supplemented with various nutrients, such as non-essential amino acids and vitamins, to enhance stem cell growth. Examples of minimal essential media include, but are not limited to, Minimal Essential Medium Eagle (MEM), Alpha MEM, Dulbecco's modified Eagle medium (DMEM), RPMI-1640 medium, 199 medium, and F12 medium. Additionally, the minimal essential media may be supplemented with additives such as horse, calf or fetal bovine serum. Alternatively, the medium can be serum free. In other cases, the growth media may contain "knockout serum replacement," referred to herein as a serum-free formulation optimized to grow and maintain undifferentiated cells, such as stem cell, in culture. KNOCKOUT^{™} serum replacement is disclosed, for example, in U.S. Patent Application No. 2002/0076747ference. Preferably, the PSCs are cultured in a fully defined and feeder free media.

Accordingly, the single cell PSCs are generally cultured in a fully defined culture medium after plating. In certain aspects, about 18-24 hours after seeding, the medium is aspirated and fresh medium, such as E8^{™} medium, is added to the culture. In certain aspects, the single cell PSCs are cultured in the fully defined culture medium for about 1, 2 or 3 days after plating. Preferably, the single cells PSCs are cultured in the fully defined culture medium for about 2 days before proceeding with the differentiation process.

In some embodiments, the medium may contain or may not contain any alternatives to serum. The alternatives to serum can include materials which appropriately contain albumin (such as lipid-rich albumin, albumin substitutes such as recombinant albumin, plant starch, dextrans and protein hydrolysates), transferrin (or other iron transporters), fatty acids, insulin, collagen precursors, trace elements, 2-mercaptoethanol, 1'-thioglycerol, or equivalents thereto. The alternatives to serum can be prepared by the method disclosed in International Publication No. WO 98/30679, for example. Alternatively, any commercially available materials can be used for more convenience. The commercially available materials include KNOCKOUT^{™} Serum Replacement (KSR), Chemically-defined Lipid concentrated (Gibco), and GLUTAMAX^{™} (Gibco).

Other culturing conditions can be appropriately defined. For example, the culturing temperature can be about 30 to 40°C, for example, at least or about 31, 32, 33, 34, 35, 36, 37, 38, 39°C but particularly not limited to them. In one embodiment, the cells are cultured at 37°C. The CO₂ concentration can be about 1 to 10%, for example, about 2 to 5%, or any range derivable therein. The oxygen tension can be at least, up to, or about 1, 2, 3, 4, 5, 6, 7, 8, 9, 10, 20%, or any range derivable therein.

### 4. Differentiation Media

### Retinal Induction Medium

After the single cell PSCs have adhered to the culture plate, the cells are preferably cultured in Retinal Induction Medium to start the differentiation process into retinal lineage cells. The Retinal Induction Medium (RIM) comprises a WNT pathway inhibitor and can result in the differentiation of PSCs to retinal lineage cells. The RIM additionally comprises a TGFβ pathway inhibitor and a BMP pathway inhibitor.

The RIM can include DMEM and F12 at about a 1:1 ratio. In exemplary methods, a WNT pathway inhibitor is included in the RIM, such as CKI-7, a BMP pathway inhibitor is included, such as LDN193189, and the TGFβ pathway inhibitor is included, such as SB431542. For example, the RIM comprises about 5 nM to about 50 nM, such as about 10 nM, of LDN193189, about 0.1 µM to about 5 µM, such as about 0.5 µM, of CKI-7, and about 0.5 µM to about 10 µM, such as about 1 µM, of SB431542. Additionally, the RIM can include knockout serum replacement, such as about 1% to about 5%, MEM non-essential amino acids (NEAA), sodium pyruvate, N-2 supplement, B-27 supplement, ascorbic acid, and insulin growth factor 1 (IGF1). Preferably, the IGF1 is animal free IGF1 (AF-IGF1) and is comprised in the RIM from about 0.1 ng/mL to about 10 ng/mL, such as about 1 ng/mL. The media is such as aspirated each day and replaced with fresh RIM. The cells are generally cultured in the RIM for about 1 to about 5 days, such as about 1, 2, 3, 4 or 5 days, such as for about 2 days to produce retinal lineage cells.

### Retinal Differentiation Medium

The retinal lineage cells can then be cultured in Retinal Differentiation Medium (RDM) for further differentiation. The RDM comprises a WNT pathway inhibitor, a BMP pathway inhibitor, a TGFβ pathway inhibitor and a MEK inhibitor. In one embodiment, the RDM comprises a WNT pathway inhibitor, such as CKI-7, a BMP pathway inhibitor, such as LDN193189, a TGFβ pathway inhibitor, such as SB431542, and a MEK inhibitor, such as PD0325901. Alternatively, the RDM can comprise a WNT pathway inhibitor, a BMP pathway inhibitor, a TGFβ pathway inhibitor and a bFGF inhibitor. Generally, the concentrations of the Wnt pathway inhibitor, BMP pathway inhibitor and TGFβ pathway inhibitor are higher in the RDM as compared to the RIM, such as about 9 to about 11 times higher, such as about 10 times higher. In exemplary methods, the RDM comprises about 50 nM to about 200 nM, such as about 100 nM of LDN193189, about 1 µM to about 10 µM, such as about 5 µM, of CKI-7, about 1 µM to about 50 µM, such as about 10 µM, of SB431542, and about 0.1 µM to about 10 µM, such as about 1 µM, 2 µM, 3 µM, 4 µM, 5 µM, 6 µM, 7 µM, 8 µM, or 9 µM of PD0325901.

Generally, the RDM comprises DMEM and F12 at about a 1:1 ratio, knockout serum replacement (*e.g.,* about 1% to about 5%, such as about 1.5%), MEM NEAA, sodium pyruvate, N-2 supplement, B-27 supplement, ascorbic acid and IGF1 (*e.g.,* about 1 ng/mL to about 50 ng/mL, such as about 10 ng/mL). In particular methods, the cells are given fresh RDM each day after aspiration of the media from the previous day. Generally, the cells are cultured in the RDM for about 2, 3, 4, 5, 6, 7, 8, 9, 10, 11, 12, 13, 14, 15, or 16 days, such as for about 7 days to derive differentiated retinal cells.

### Retinal Medium

Next, the differentiated retinal cells can be even further differentiated by culturing the cells in Retinal Medium (RM). The Retinal Medium comprises Activin A and can additionally comprise Nicotinamide. The RM can comprise about 50 to about 200 ng/mL, such as about 100 ng/mL, of ACTIVIN A, and about 1 mM to about 50 mM, such as about 10 mM, of nicotinamide. Alternatively, the RM can comprise other TGF-β pathway activators such as GDF1 and/or WNT pathway activators such as CHIR99021, WAY-316606, IQ1, QS11, SB-216763, BIO (6-bromoindirubin-3'-oxime), or 2-amino-4-[3,4-(methylenedioxy)benzylamino]-6-(3-methoxyphenyl) pyrimidine. Alternatively, the RM can additionally comprise WNT3a.

The RM can include DMEM and F12 at about a 1:1 ratio, knockout serum replacement at about 1% to about 5%, such as about 1.5%, MEM non-essential amino acids (NEAA), sodium pyruvate, N-2 supplement, B-27 supplement, and ascorbic acid. The medium can be changed daily with room temperature RM. The cells are generally cultured in the RM for about 8, 9, 10, 11, 12, 13, 14, 15, 16 or 17 days, such as for about 10 days to derive differentiating RPE cells.

### RPE-Maturation Medium

For further differentiation of the RPE cells, the cells are preferably cultured in RPE Maturation Medium (RPE-MM). Exemplary RPE-MM media are shown in Table 3. The RPE-Maturation Medium can comprise about 100 µg/mL to about 300 µg/mL, such as about 250 µg/mL, of taurine, about 10 µg/L to about 30 µg/L, such as about 20 µg/L, of hydrocortisone and about 0.001 µg/L to about 0.1 µg/L, such as about 0.013 µg/L, of triiodothyronine. Additionally, the RPE-MM can comprise MEM Alpha, N-2 supplement, MEM non-essential amino acids (NEAA), and sodium pyruvate, and fetal bovine serum (or KnockOut^{™} Serum Replacement) (*e.g.,* about 0.5% to about 10%, such as about 1% to about 5%). The medium can be changed every other day with room temperature RPE-MM. The cells are generally cultured in RPE-MM for about 5 to about 10 days, such as about 5 days. The cells can then be dissociated, such as with a cell dissociation enzyme, reseeded, and cultured for an additional period of time, such as an additional about 5 to about 30 days, such as about 15 to 20 days, for further differentiation into RPE cells. In further embodiments, the RPE-MM does not include a WNT pathway inhibitor. RPE cells can be cryopreserved at this stage.

### b. Maturation of RPE Cells

The RPE cells can then be cultured in the RPE-MM for a continued period of time for maturation. In some embodiments, the RPE cells are grown in wells, such as a flask, multi-layer flask, 6-well, 12-well, 24-well, or 10 cm plate. The RPE cells can be maintained in RPE medium for about four to about ten weeks, such as for about six to eight weeks, such as for six, seven, or eight weeks. In exemplary methods for the continued maturation of the RPE cells, the cells can be dissociated in a cell dissociated enzyme such as TRYPLE^{™} and reseeded on a degradable scaffold assembly such as in a specialized SNAPWELL^{™} design for about one to ten weeks, such as five weeks in RPE-MM. The RPE-MM can comprise a bFGF inhibitor or a MEK inhibitor. The methods for culturing RPE cells on a degradable scaffold are taught and described in PCT Publication No. WO 2014/121077. Briefly, the main components of this method are a CORNING^{®} COSTAR^{®} SNAPWELL^{™} plate, a bioinert O-ring, and a biodegradable scaffold. SNAPWELL^{™} plates provide the structure and platform for the biodegradable scaffolds. The microporous membrane that creates an apical and basal side is ideal for providing support to the scaffold as well as isolating the distinct sides of the polarized layer of cells. The ability of the SNAPWELL^{™} insert to detach the membrane allows the support ring of the insert to be used as an anchor for the scaffold. The resulting differentiated, polarized, and confluent monolayers of functional RPE cells can be cryopreserved at this stage *(e.g.,* in xenofree CS10 medium).

In some embodiments, mature RPE cells can be further developed into functional RPE cell monolayers that behave as intact RPE tissue by continued culture in the RPE-MM with additional chemicals or small molecules that promote RPE maturation. For example, these small molecules are primary cilium inducers such as prostaglandin E2 (PGE2) or aphidicolin. The PGE2 may be added to the medium at a concentration of about 25 µM to about 250 µM , such as about 50 µM to about 100 µM. Alternatively, the RPE-MM can comprise canonical WNT pathway inhibitors. Exemplary canonical WNT pathway inhibitors are N-(6-Methyl-2-benzothiazolyl)-2-[(3,4,6,7-tetrahydro-4-oxo-3-phenylthieno[3,2-d]pyrimidin-2-yl)thio]-acetamide (IWP2) or 4-(1,3,3a,4,7,7a-Hexahydro-1,3-dioxo-4,7-methano-2H-isoindol-2-yl)-N-8-quinolinyl-Benzamide (endo-IWR1). The cells are can be cultured in this medium for an additional period of time, such as an additional about one week to about five weeks, such as about another two to four weeks to obtain mature and functional RPE cell monolayers. Thus, the presently disclosed methods provide mature RPE cells from single cell suspensions of pluripotent cells that can be consistently reproduced at a large scale for clinical applications.

### B. Photoreceptor Cells

PRP can be produced in the methods disclosed herein. The cells in the retina that are directly sensitive to light are the photoreceptor cells. Photoreceptors are photosensitive neurons in the outer part of the retina and can be either rods or cones. In the process of phototransduction, the photoreceptor cells convert incident light energy focused by the cornea and lens to electric signals which are ultimately sent via the optic nerve to the brain. Vertebrates have two types of photoreceptor cells including cones and rods. Cones are adapted to detect fine detail, central and color vision and function well in bright light. Rods are responsible for peripheral and dim light vision. Neural signals from the rods and cones undergo processing by other neurons of the retina.

Photoreceptors can express markers such as OTX2, CRX, PRDM1 (BLIMP1), NEUROD1, RCVRN, TUBB3 and L1CAM (CD171). Photoreceptors express several proteins that can serve as markers for detection by the use of methodologies, such as immunocytochemistry, western blot analysis, flow cytometry, or enzyme-linked immunoassay (ELISA). For example, one characteristic PRP-marker is RCVRN. PRP cells may not express (at any detectable level) the embryonic stem cells markers OCT-4, NANOG or REX-1. Specifically, expression of these genes is approximately 100-1000 fold lower in PR/PRP cells than in ES cells or iPSC cells, when assessed by quantitative RT-PCR.

Photoreceptor cell markers may be detected at the mRNA level, for example, by reverse transcriptase polymerase chain reaction (RT-PCR), Northern blot analysis, microarray, or RNA-sequencing including single-cell RNA sequencing dot-blot hybridization analysis using sequence-specific primers in standard amplification methods using publicly available sequence data (GENBANK^{®}). Expression of tissue-specific markers as detected at the protein or mRNA level is considered positive if the level is at least or about 2-, 3-, 4-, 5-, 6-, 7-, 8-, or 9-fold, and more particularly more than 10-, 20-, 30, 40-, 50-fold or higher above that of a control cell, such as an undifferentiated pluripotent stem cell or other unrelated cell type.

Dysfunction, injury and loss of photoreceptor cells are factors of many eye diseases and disorders including age-related macular degeneration (AMD), hereditary macular degenerations including Best disease, Stargardt disease and choroideremia, retinitis pigmentosa, and other forms of inherited retinal diseases and acquired retinal dysfunctions, diseases, and injuries. A potential treatment for such diseases is the transplantation of PRP or photoreceptor cells into the retina of those in need of such treatment. It is speculated that the replenishment of PRP or PR by their transplantation may delay, halt or reverse degradation, improve retinal function and prevent blindness stemming from such conditions. However, obtaining PRP or PR directly from human donors and embryos is a challenge.

In some embodiments, methods are provided for producing PRP cells from an essentially single cell suspension of PSCs such as human iPSCs. In some embodiments, the PSCs are cultured to pre-confluence. In certain aspects, the PSCs are dissociated by incubation with a cell dissociation solution or enzyme, such as exemplified by Versene, Trypsin, ACCUTASE^{™} or TRYPLE^{™}. PSCs can also be dissociated into an essentially single cell suspension by pipetting.

In addition, Blebbistatin (*e.g.,* about 2.5 µM) can be added to the medium to increase PSC survival after dissociation into single cells while the cells are not adhered to a culture vessel. A ROCK inhibitor instead of Blebbistatin may alternatively be used to increase PSC survival after dissociation into single cells.

Once a single cell suspension of PSCs is obtained, the cells are generally seeded in an appropriate culture vessel, such as a tissue culture plate, such as a flask, multi-layer flask, 6-well, 12-well, 24-well, 96-well or 10 cm plate. A culture vessel used for culturing the cell(s) can include, but is particularly not limited to: flask, flask for tissue culture, dish, Petri dish, dish for tissue culture, multi dish, micro plate, micro-well plate, multi plate, multi-well plate, micro slide, chamber slide, tube, tray, CELLSTACK^{®} Chambers, culture bag, and roller bottle, as long as it is capable of culturing the stem cells therein. The cells may be cultured in a volume of at least or about 0.2, 0.5, 1, 2, 5, 10, 20, 30, 40, 50 ml, 100 ml, 150 ml, 200 ml, 250 ml, 300 ml, 350 ml, 400 ml, 450 ml, 500 ml, 550 ml, 600 ml, 800 ml, 1000 ml, 1500 ml, or any range derivable therein, depending on the needs of the culture. In a certain embodiment, the culture vessel may be a bioreactor, which may refer to any device or system *ex vivo* that supports a biologically active environment such that cells can be propagated. The bioreactor may have a volume of at least or about 2, 4, 5, 6, 8, 10, 15, 20, 25, 50, 75, 100, 150, 200, 500 liters, 1, 2, 4, 6, 8, 10, 15 cubic meters, or any range derivable therein.

In certain aspects, the PSCs, such as iPSCs, are plated at a cell density appropriate for efficient differentiation. Generally, the cells are plated at a cell density of about 1,000 to about 75,000 cells/cm², such as of about 5,000 to about 40,000 cells/cm². In a 6 well plate, the cells may be seeded at a cell density of about 50,000 to about 400,000 cells per well. In exemplary methods, the cells are seeded at a cell density of about 100,000, about 150,000, about 200,000, about 250,000, about 300,000 or about 350,000 cells per well, such as about 50,000 cells per well.

The PSCs, such as iPSCs, are generally cultured on culture plates coated by one or more cellular adhesion proteins to promote cellular adhesion while maintaining cell viability. For example, preferred cellular adhesion proteins include extracellular matrix proteins such as vitronectin, laminin, collagen, and/or fibronectin, which may be used to coat a culturing surface as a means of providing a solid support for pluripotent cell growth. The term "extracellular matrix (ECM)" is recognized in the art. Its components can include, but are not limited to, one or more of the following proteins: fibronectin, laminin, vitronectin, tenascin, entactin, thrombospondin, elastin, gelatin, collagen, fibrillin, merosin, anchorin, chondronectin, link protein, bone sialoprotein, osteocalcin, osteopontin, epinectin, hyaluronectin, undulin, epiligrin, and kalinin. Other ECM components may include synthetic peptides for adhesion (*e.g.,* RGD or IKVAV motifs), synthetic hydrogels (*e.g.,* PEG, PLGA, etc.) or natural hydrogels, such as alginate. In exemplary methods, the PSCs are grown on culture plates coated with vitronectin. In some embodiments, the cellular adhesion proteins are human proteins.

The extracellular matrix proteins may be of natural origin and purified from human or animal tissues or, alternatively, the ECM proteins may be genetically engineered recombinant proteins or synthetic in nature. The ECM proteins may be a whole protein or in the form of peptide fragments, native or engineered. Examples of ECM protein that may be useful in the matrix for cell culture include laminin, collagen I, collagen IV, fibronectin and vitronectin. In some embodiments, the matrix composition is xeno-free. For example, in the xeno-free matrix to culture human cells, matrix components of human origin may be used, wherein any non-human animal components may be excluded.

In some aspects, the total protein concentration in the matrix composition may be about 1 ng/mL to about 1 mg/mL. In some preferred embodiments, the total protein concentration in the matrix composition is about 1 µg/mL to about 300 µg/mL. In more preferred embodiments, the total protein concentration in the matrix composition is about 5 µg/mL to about 200 µg/mL.

Cells, such as PR/PRP cells or PSCs, can be cultured with the nutrients necessary to support the growth of each specific population of cells. Generally, the cells are cultured in growth media including a carbon source, a nitrogen source and a buffer to maintain pH. The medium can also contain fatty acids or lipids, amino acids (such as non-essential amino acids), vitamin(s), growth factors, cytokines, antioxidant substances, pyruvic acid, buffering agents, pH indicators, and inorganic salts. An exemplary growth medium contains a minimal essential media, such as Dulbecco's Modified Eagle's medium (DMEM) or ESSENTIAL 8^{™} (E8^{™}) medium, supplemented with various nutrients, such as non-essential amino acids and vitamins, to enhance stem cell growth. Examples of minimal essential media include, but are not limited to, Minimal Essential Medium Eagle (MEM) Alpha medium, Dulbecco's modified Eagle medium (DMEM), RPMI-1640 medium, 199 medium, and F12 medium. Additionally, the minimal essential media may be supplemented with additives such as horse, calf or fetal bovine serum. Alternatively, the medium can be serum free. In other cases, the growth media may contain "knockout serum replacement," referred to herein as a serum-free formulation optimized to grow and maintain undifferentiated cells, such as stem cell, in culture. KNOCKOUT^{™} serum replacement is disclosed, for example, in U.S. Patent Application No. 2002/0076747. Preferably, the PSCs are cultured in a fully-defined and feeder-free media.

Accordingly, the single cell PSCs are generally cultured in a fully defined culture medium after plating. In certain aspects, about 18-24 hours after seeding, the medium is aspirated and fresh medium, such as E8^{™} medium, is added to the culture. In certain aspects, the single cell PSCs are cultured in the fully defined culture medium for about 1, 2 or 3 days after plating. Preferably, the single cells PSCs are cultured in the fully defined culture medium for about 2 days before proceeding with the differentiation process.

In some embodiments, the medium may contain or may not contain any alternatives to serum. The alternatives to serum can include materials which appropriately contain albumin (such as lipid-rich albumin, albumin substitutes such as recombinant albumin, plant starch, dextrans and protein hydrolysates), transferrin (or other iron transporters), fatty acids, insulin, collagen precursors, trace elements, 2-mercaptoethanol, 3'-thioglycerol, or equivalents thereto. The alternatives to serum can be prepared by the method disclosed in International Publication No. WO 98/30679, for example. Alternatively, any commercially available materials can be used for more convenience. The commercially available materials include KNOCKOUT^{™} Serum Replacement (KSR), Chemically-defined Lipid concentrated (Gibco), and GLUTAMAX^{™} (Gibco).

Other culturing conditions can be appropriately defined. For example, the culturing temperature can be about 30 to 40°C, for example, at least or about 31, 32, 33, 34, 35, 36, 37, 38, 39°C but particularly not limited to them. In one embodiment, the cells are cultured at 37°C. The CO₂ concentration can be about 1 to 10%, for example, about 2 to 5%, or any range derivable therein. The oxygen tension can be at least, up to, or about 1, 2, 3, 4, 5, 6, 7, 8, 9, 10, 20%, or any range derivable therein.

### 2. Differentiation Media

### Retinal Maturation Medium

The differentiated retinal cells cultured in RDM as described above can be further differentiated and expanded by culturing the cells in Retinal Maturation Medium (RM) to produce RPCs. The RM may comprise nicotinamide. The RM can comprise about 1 mM to about 50 mM, such as about 10 mM, of nicotinamide. The RM may further comprise ascorbic acid, such as 50-500 µm, particularly about 100-300 µm, such as about 200 µm. Preferably, the RM is free of or essentially free of Activin A. Exemplary RM media are shown in Table 1. The RM *(e.g.,* RM2) may further comprise a γ-secretase inhibitor, such as DAPT, basic FGF, and/or a TGFβ pathway inhibitor, such as SB431542.

The RM can include DMEM and F12 at about a 1:1 ratio, knockout serum replacement at about 1% to about 5%, such as about 1.5%, MEM non-essential amino acids (NEAA), sodium pyruvate, N-2 supplement, B-27 supplement, and ascorbic acid. The medium can be changed daily with room temperature RM. The cells are generally cultured in the RM for about 8, 9, 10, 11, 12, 13, 14, 15, 16 or 17 days, such as for about 10 days to derive expanded RPCs.

### PRP Maturation Medium (PM)

The PRPs may be matured in PRP maturation medium (PM). Exemplary PM medium is shown in Table 1. The PM medium comprises ascorbic acid, nicotinamide, and a γ-secretase inhibitor, such as DAPT (*e.g.,* about 1 µM to about 10 µM, such as about 5 µM of DAPT). The PM *(e.g.,* PM2) may also comprise a CDK inhibitor, such as a CDK4/6 inhibitor, such as PD0332991 (*e.g.,* about 1 µM to about 50 µM, such as about 10 µM of PD0332991).

The PM Medium can include DMEM and F12 at about a 1:1 ratio, knockout serum replacement at about 1% to about 5%, such as about 1.5%, MEM non-essential amino acids (NEAA), sodium pyruvate, N-2 supplement, B-27 supplement, and ascorbic acid. The medium can be changed daily with room temperature PM Medium. The cells are generally cultured in the PM medium for about 4, 5, 6, 7, 8, 9, 10, 11, 12, 13, 14, 15, 16, 17, 18, 19, or 20 days, such as for about 10 days to derive mature PRP cells.

**Table 1: Exemplary Medium Components**

| **Essential 8 Medium** | | | |
|---|---|---|---|
| **Component** | **Vendor** | **Cat#** | **Final Conc.** |
| Essential 8^{™} Basal Medium | Thermo Fisher | A1517001 | 98% |
| Essential 8^{™} Supplement | Thermo Fisher | | 2% |

| **Essential 8 Thawing Medium** | | | |
|---|---|---|---|
| **Component** | **Vendor** | **Cat#** | **Final Conc.** |
| Complete Essential 8^{™} Medium | Thermo Fisher | As prepared above | 100% |
| Rho Kinase Inhibitor (H1152) | Millipore Sigma | 555550 | 1 µM |

| **Essential 8 Plating Medium** | | | |
|---|---|---|---|
| **Component** | **Vendor** | **Cat#** | **Final Conc.** |
| Complete Essential 8^{™} Medium | Thermo Fisher | As prepared above | 100% |
| Blebbistatin | Millipore Sigma | B0560 | 2.5 µM |

| **Retinal Induction Medium (RIM)** | | | |
|---|---|---|---|
| **Component** | **Vendor** | **Cat#** | **Final Conc.** |
| DMEM/F12 | Thermo Fisher | 11330-032 | 99% |
| CTS^{™} KnockOut^{™} SR XenoFree | Thermo Fisher | A1099201 | 1.50% |
| MEM non-essential AA | Thermo Fisher | 11140 | 0.1mM |
| Sodium Pyruvate | Thermo Fisher | 11360-070 | 1mM |
| CTS^{™} N-2 Supplement | Thermo Fisher | A13707-01 | 1% |
| B-27^{®} Supplement (+VitA) | Thermo Fisher | 17504-044 | 2% |
| Ascorbic Acid | Millipore Sigma | A4544 | 200µM |
| LDN-193189 | Stemgent | 04-0074 | 10nM |
| SB 431542 | R&D Systems | 1614/10 | 1.0µM |
| CKI-7 Dihydrochloride | Millipore Sigma | C0742 | 0.5µM |
| AF-IGF-1 | R&D Systems | AFL291 | 1ng/ml |

| **Retinal Differentiation Medium #1 (RD1)** | | | |
|---|---|---|---|
| **Component** | **Vendor** | **Cat#** | **Final Conc.** |
| DMEM/F12 | Thermo Fisher | 11330-032 | 99% |
| CTS^{™} KnockOut^{™} SR XenoFree | Thermo Fisher | A1099201 | 1.50% |
| MEM non-essential AA | Thermo Fisher | 11140 | 0.1mM |
| Sodium Pyruvate | Thermo Fisher | 11360-070 | 1mM |
| N-2 Supplement | Thermo Fisher | A13707-01 | 1% |
| B-27^{®} Supplement (+VitA) | Thermo Fisher | 17504-044 | 2% |
| Ascorbic Acid | Millipore Sigma | A4544 | 200µM |
| LDN-193189 | Stemgent | 04-0074 | 100nM |
| SB 431542 | R&D Systems | 1614/10 | 10µM |
| CKI-7 Dihydrochloride | Millipore Sigma | C0742 | 5µM |
| AF-IGF-1 | R&D Systems | AFL291 | 10ng/ml |
| PD0325901 | Stemgent | 04-0006 | 1µM |

| **Retinal Differentiation Medium #2 (RD2)** | | | |
|---|---|---|---|
| **Component** | **Vendor** | **Cat#** | **Final Conc.** |
| DMEM/F12 | Thermo Fisher | 11330-032 | 99% |
| CTS^{™} KnockOut^{™} SR XenoFree | Thermo Fisher | A1099201 | 1.50% |
| MEM non-essential AA | Thermo Fisher | 11140 | 0.1mM |
| Sodium Pyruvate | Thermo Fisher | 11360-070 | 1mM |
| N-2 Supplement | Thermo Fisher | A13707-01 | 1% |
| B-27^{®} Supplement (+VitA) | Thermo Fisher | 17504-044 | 2% |
| Ascorbic Acid | Millipore Sigma | A4544 | 200µM |
| SB 431542 | R&D Systems | 1614/10 | 10µM |
| CKI-7 Dihydrochloride | Millipore Sigma | C0742 | 5µM |
| AF-IGF-1 | R&D Systems | AFL291 | 10ng/ml |
| PD0325901 | Stemgent | 04-0006 | 1µM |

| **Retinal Maturation Medium #1 (RM1)** | | | |
|---|---|---|---|
| **Component** | **Vendor** | **Cat#** | **Final Conc.** |
| DMEM/F12 | Thermo Fisher | 11330-032 | 99% |
| CTS^{™} KnockOut^{™} SR XenoFree Kit | Thermo Fisher | A1099201 | 1.50% |
| MEM non-essential AA | Thermo Fisher | 11140 | 0.1mM |
| Sodium Pyruvate | Thermo Fisher | 11360-070 | 1mM |
| CTS^{™} N-2 Supplement | Thermo Fisher | A13707-01 | 1% |
| B-27^{®} Supplement (+VitA) | Thermo Fisher | 17504-044 | 2% |
| Ascorbic Acid | Millipore Sigma | A4544 | 200µM |
| Nicotinamide | Millipore Sigma | N0636 | 10mM |

| **Retinal Maturation Medium #2 (RM2)** | | | |
|---|---|---|---|
| **Component** | **Vendor** | **Cat#** | **Final Conc.** |
| DMEM/F12 | Thermo Fisher | 11330-032 | 99% |
| CTS^{™} KnockOut^{™} SR XenoFree Kit | Thermo Fisher | A1099201 | 1.50% |
| MEM non-essential AA | Thermo Fisher | 11140 | 0.1mM |
| Sodium Pyruvate | Thermo Fisher | 11360-070 | 1mM |
| CTS^{™} N-2 Supplement | Thermo Fisher | A13707-01 | 1% |
| B-27^{®} Supplement (+VitA) | Thermo Fisher | 17504-044 | 2% |
| Ascorbic Acid | Millipore Sigma | A4544 | 200µM |
| Nicotinamide | Millipore Sigma | N0636 | 10mM |
| basic FGF | R&D Systems | AFL233 | 10-100ng/mL |
| SB 431542 | R&D Systems | 1614/10 | 10µM |

| **PRP Maturation Medium #1 (PM1)** | | | |
|---|---|---|---|
| **Component** | **Vendor** | **Cat#** | **Final Conc.** |
| DMEM/F12 | Thermo Fisher | 11330-032 | 99% |
| CTS^{™} KnockOut^{™} SR XenoFree Kit | Thermo Fisher | A1099201 | 1.50% |
| MEM non-essential AA | Thermo Fisher | 11140 | 0.1mM |
| Sodium Pyruvate | Thermo Fisher | 11360-070 | 1mM |
| CTS^{™} N-2 Supplement | Thermo Fisher | A13707-01 | 1% |
| B-27^{®} Supplement (+VitA) | Thermo Fisher | 17504-044 | 2% |
| Ascorbic Acid | Millipore Sigma | A4544 | 200µM |
| Nicotinamide | Millipore Sigma | N0636 | 10mM |
| DAPT | Millipore Sigma | D5942 | 5µM |

| **PRP Maturation Medium #1 (PM2)** | | | |
|---|---|---|---|
| **Component** | **Vendor** | **Cat#** | **Final Conc.** |
| DMEM/F12 | Thermo Fisher | 11330-032 | 99% |
| CTS^{™} KnockOut^{™} SR XenoFree Kit | Thermo Fisher | A1099201 | 1.50% |
| MEM non-essential AA | Thermo Fisher | 11140 | 0.1mM |
| Sodium Pyruvate | Thermo Fisher | 11360-070 | 1mM |
| CTS^{™} N-2 Supplement | Thermo Fisher | A13707-01 | 1% |
| B-27^{®} Supplement (+VitA) | Thermo Fisher | 17504-044 | 2% |
| Ascorbic Acid | Millipore Sigma | A4544 | 200µM |
| Nicotinamide | Millipore Sigma | N0636 | 10mM |
| DAPT | Millipore Sigma | D5942 | 5µM |
| PD0332991 | Tocris | 4786 | 10 µM |

| **MACS Buffer** | | | |
|---|---|---|---|
| **Component** | **Vendor** | **Cat#** | **Final Conc.** |
| DPBS (without calcium and magnesium) | Thermo Fisher | A1285601 | 98% |
| Human Serum Albumin | Irvine Scientific | 9988-100ML | 2% |
| EDTA Solution OmniPur, 0.5 Molar, pH 8.0 | EMD Millipore | 4055-100ML | 2mM |
| Benzonase nuclease | EMD Millipore | 1016970001 | 50 U/mL |

| **Post-thaw Medium #1 (PT1)** | | | |
|---|---|---|---|
| **Component** | **Vendor** | **Cat#** | **Final Conc.** |
| Neurobasal CTS Grade | Thermo Fisher | A13712-01 | 99% |
| CTS^{™} N-2 Supplement | Thermo Fisher | A13707-01 | 1% |
| Glutamax | Thermo Fisher | 35050-061 | 1% |
| Y-27632 | Tocris | 1254/10 | 10 µM |

| **Post-thaw Medium #2 (PT2)** | | | |
|---|---|---|---|
| **Component** | **Vendor** | **Cat#** | **Final Conc.** |
| DMEM/F12 | Thermo Fisher | 11330-032 | 99% |
| CTS^{™} KnockOut^{™} SR XenoFree Kit | Thermo Fisher | A1099201 | 1.50% |
| MEM non-essential AA | Thermo Fisher | 11140 | 0.1mM |
| Sodium Pyruvate | Thermo Fisher | 11360-070 | 1mM |
| CTS^{™} N-2 Supplement | Thermo Fisher | A13707-01 | 1% |
| B-27^{®} Supplement (+VitA) | Thermo Fisher | 17504-044 | 2% |
| Ascorbic Acid | Millipore Sigma | A4544 | 200µM |
| Nicotinamide | Millipore Sigma | N0636 | 10mM |
| Y-27632 (optional) | Tocris | 1254/10 | 10 µM |

### RPE-MM

| **Component** | **Vendor** | **Cat#** | **Final Conc.** |
|---|---|---|---|
| MEM Alpha | Thermo Fisher | 12571-063 | 94% |
| Fetal Bovine Serum | Hyclone | SH30071.03 | 5% |
| CTS^{™} N-2 Supplement | Thermo Fisher | A13707-01 | 1% |
| MEM non-essential AA | Thermo Fisher | 11140 | 0.1mM |
| Sodium Pyruvate | Thermo Fisher | 11360-070 | 1mM |
| Taurine | Sigma | T4571 | 250 µg/ml |
| Hydrocortisone | Sigma | H6909 | 55.2nM (20 µg/L) |
| 3,3',5-Triiodo-L-thyronine | Sigma | T5516 | 0.013µg/L |

### RPE-MM XF

| **Component** | **Vendor** | **Cat#** | **Final Conc.** |
|---|---|---|---|
| MEM Alpha | Thermo Fisher | 12571-063 | 94% |
| CTS^{™} KnockOut^{™} SR XenoFree Kit | Thermo Fisher | A1099201 | 5% |
| CTS^{™} N-2 Supplement | Thermo Fisher | A13707-01 | 1% |
| MEM non-essential AA | Thermo Fisher | 11140 | 0.1mM |
| Sodium Pyruvate | Thermo Fisher | 11360-070 | 1mM |
| Taurine | Sigma | T4571 | 250 µg/ml |
| Hydrocortisone | Sigma | H6909 | 55.2nM (20 µg/L) |
| 3,3',5-Triiodo-L-thyronine | Sigma | T5516 | 13 ng/L |

### RPE-MM + PGE2

| **Component** | **Vendor** | **Cat#** | **Final Conc.** |
|---|---|---|---|
| MEM Alpha | Thermo Fisher | 12571-063 | 94% |
| Fetal Bovine Serum | Hyclone | SH30071.03 | 5% |
| CTS^{™} N-2 Supplement | Thermo Fisher | A13707-01 | 1% |
| MEM non-essential AA | Thermo Fisher | 11140 | 0.1mM |
| Sodium Pyruvate | Thermo Fisher | 11360-070 | 1mM |
| Taurine | Sigma | T4571 | 250 µg/ml |
| Hydrocortisone | Sigma | H6909 | 55.2nM (20 µg/L) |
| 3,3',5-Triiodo-L-thyronine | Sigma | T5516 | 13ng/L |
| PGE2 | Tocris | 2296 | 100µM |

### RPE-MM XF + PGE2

| **Component** | **Vendor** | **Cat#** | **Final Conc.** |
|---|---|---|---|
| MEM Alpha | Thermo Fisher | 12571-063 | 94% |
| CTS^{™} KnockOut^{™} SR XenoFree Kit | Thermo Fisher | A1099201 | 5% |
| CTS^{™} N-2 Supplement | Thermo Fisher | A13707-01 | 1% |
| MEM non-essential AA | Thermo Fisher | 11140 | 0.1mM |
| Sodium Pyruvate | Thermo Fisher | 11360-070 | 1mM |
| Taurine | Sigma | T4571 | 250 µg/ml |
| Hydrocortisone | Sigma | H6909 | 55.2nM (20 µg/L) |
| 3,3',5-Triiodo-L-thyronine | Sigma | T5516 | 13 ng/L |
| PGE2 | Tocris | 2296 | 100µM |

### RPE-MM Thaw

| **Component** | **Vendor** | **Cat#** | **Final Conc.** |
|---|---|---|---|
| MEM Alpha | Thermo Fisher | 12571-063 | 94% |
| Fetal Bovine Serum | Hyclone | SH30071.03 | 5% |
| CTS^{™} N-2 Supplement | Thermo Fisher | A13707-01 | 1% |
| MEM non-essential AA | Thermo Fisher | 11140 | 0.1mM |
| Sodium Pyruvate | Thermo Fisher | 11360-070 | 1mM |
| Taurine | Sigma | T4571 | 250 µg/ml |
| Hydrocortisone | Sigma | H6909 | 55.2nM (20 µg/L) |
| 3,3',5-Triiodo-L-thyronine | Sigma | T5516 | 0.013µg/L |
| Y-27632 | Tocris | 1254 | 10µM |

### RPE-MM XF Thaw

| **Component** | **Vendor** | **Cat#** | **Final Conc.** |
|---|---|---|---|
| MEM Alpha | Thermo Fisher | 12571-063 | 94% |
| CTS^{™} KnockOut^{™} SR XenoFree Kit | Thermo Fisher | A1099201 | 5% |
| CTS^{™} N-2 Supplement | Thermo Fisher | A13707-01 | 1% |
| MEM non-essential AA | Thermo Fisher | 11140 | 0.1mM |
| Sodium Pyruvate | Thermo Fisher | 11360-070 | 1mM |
| Taurine | Sigma | T4571 | 250 µg/ml |
| Hydrocortisone | Sigma | H6909 | 55.2nM (20 µg/L) |
| 3,3',5-Triiodo-L-thyronine | Sigma | T5516 | 13 ng/L |
| Y-27632 | Tocris | 1254 | 10µM |

In addition, Blebbistatin (*e.g*., about 2.5 µM) can be added to the medium to increase PR/PRP survival and maintain purity by promoting aggregate formation. A ROCK inhibitor instead of Blebbistatin may alternatively be used to increase PR/PRP survival after dissociation into single cells, such as by using TRYPLE^{™}.

### C. Cryopreservation of Cells

The RPE or PR/PRP cells produced by the methods disclosed herein can be cryopreserved, see for example, PCT Publication No. 2012/149484 A2. The cells can be cryopreserved with or without a substrate. In several embodiments, the storage temperature ranges from about -50°C to about -60°C, about -60°C to about -70°C, about -70°C to about - 80°C, about -80°C to about -90°C, about -90°C to about - 100°C, and overlapping ranges thereof. In some embodiments, lower temperatures are used for the storage (*e.g*., maintenance) of the cryopreserved cells. In several embodiments, liquid nitrogen (or other similar liquid coolant) is used to store the cells. In further embodiments, the cells are stored for greater than about 6 hours. In additional embodiments, the cells are stored about 72 hours. In several embodiments, the cells are stored 48 hours to about one week. In yet other embodiments, the cells are stored for about 1, 2, 3, 4, 5, 6, 7, or 8 weeks. In further embodiments, the cells are stored for 1, 2, 3, 4, 5, 67, 8, 9, 10, 11 or 12 months. The cells can also be stored for longer times. The cells can be cryopreserved separately or on a substrate, such as any of the substrates disclosed herein.

In some embodiments, additional cryoprotectants can be used. For example, the cells can be cryopreserved in a cryopreservation solution comprising one or more cryoprotectants, such as DM80, serum albumin, such as human or bovine serum albumin. In certain embodiments, the solution comprises about 1 %, about 1.5%, about 2%, about 2.5%, about 3%, about 4%, about 5%, about 6%, about 7%·, about 8%, about 9%, or about 10% DMSO. In other embodiments, the solution comprises about 1% to about 3%, about 2% to about 4%, about 3% to about 5%, about 4% to about 6%, about 5% to about 7%, about 6% to about 8%, about 7% to about 9%, or about 8%· to about 10% dimethylsulfoxide (DMSO) or albumin. In a specific embodiment, the solution comprises 2.5% DMSO. In another specific embodiment, the solution comprises 10% DMSO.

Cells may be cooled, for example, at about 1° C minute during cryopreservation. In some embodiments, the cryopreservation temperature is about -80° C to about -180° C, or about -125° C to about -140° C. In some embodiments, the cells are cooled to 4 °C prior to cooling at about 1 °C/minute. Cryopreserved cells can be transferred to vapor phase of liquid nitrogen prior to thawing for use. In some embodiments, for example, once the cells have reached about -80° C, they are transferred to a liquid nitrogen storage area. Cryopreservation can also be done using a controlled-rate freezer. Cryopreserved cells may be thawed, e.g., at a temperature of about 25° C to about 40° C, and typically at a temperature of about 37° C.

### D. Inhibitors

### WNT Pathway Inhibitors

WNT is a family of highly conserved secreted signaling molecules that regulate cell-to-cell interactions and are related to the Drosophila segment polarity gene, wingless. In humans, the WNT family of genes encodes 38 to 43 kDa cysteine rich glycoproteins. The WNT proteins have a hydrophobic signal sequence, a conserved asparagine-linked oligosaccharide consensus sequence (see *e.g.,* Shimizu et al Cell Growth Differ 8: 1349-1358 (1997)) and 22 conserved cysteine residues. Because of their ability to promote stabilization of cytoplasmic beta-catenin, WNT proteins can act as transcriptional activators and inhibit apoptosis. Overexpression of particular WNT proteins has been shown to be associated with certain cancers.

A WNT inhibitor (also referred to as a WNT pathway inhibitor) herein refers to WNT inhibitors in general. Thus, a WNT inhibitor refers to any inhibitor of a member of the WNT family proteins including Wnt1, Wnt2, Wnt2b, Wnt3, Wnt4, Wnt5A, Wnt6, Wnt7A, Wnt7B, Wnt8A, Wnt9A, Wnt10a, Wnt11, and Wnt16. Certain embodiments of the present methods concern a WNT inhibitor in the differentiation medium. Examples of suitable WNT inhibitors, already known in the art, include N-(2-Aminoethyl)-5-chloroisoquinoline-8-sulphonamide dihydrochloride (CKI-7), N-(6-Methyl-2-benzothiazolyl)-2-[(3,4,6,7-tetrahydro-4-oxo-3-phenylthieno[3,2-d]pyrimidin-2-yl)thio]-acetamide (IWP2), N-(6-Methyl-2-benzothiazolyl)-2-[(3,4,6,7-tetrahydro-3-(2-methoxyphenyl)-4-oxothieno[3,2-d]pyrimidin-2-yl)thio]-acetamide (IWP4), 2-Phenoxybenzoic acid-[(5-methyl-2-furanyl)methylene]hydrazide (PNU 74654) 2,4-diamino-quinazoline, quercetin, 3,5,7,8-Tetrahydro-2-[4-(trifluoromethyl)phenyl]-4H-thiopyrano[4,3-d]pyrimidin-4-one (XAV939), 2,5-Dichloro-N-(2-methyl-4-nitrophenyl)benzenesulfonamide (FH 535), N-[4-[2-Ethyl-4-(3-methylphenyl)-5-thiazolyl]-2-pyridinyl]benzamide (TAK 715), Dickkopf-related protein one (DKK1), and Secreted frizzled-related protein (SFRP1) 1. In addition, inhibitors of WNT can include antibodies to, dominant negative variants of, and siRNA and antisense nucleic acids that suppress expression of WNT. Inhibition of WNT can also be achieved using RNA-mediated interference (RNAi).

### BMP Pathway Inhibitors

Bone morphogenic proteins (BMPs) are multi-functional growth factors that belong to the transforming growth factor beta (TGFβ) superfamily. BMPs are considered to constitute a group of pivotal morphogenetic signals, orchestrating architecture through the body. The important functioning of BMP signals in physiology is emphasized by the multitude of roles for dysregulated BMP signaling in pathological processes.

BMP pathway inhibitors (also referred to herein as BMP inhibitors) may include inhibitors of BMP signaling in general or inhibitors specific for BMP1, BMP2, BMP3, BMP4, BMP5, BMP6, BMP7, BMP8a, BMP8b, BMP10 or BMP15. Exemplary BMP inhibitors include 4-(6-(4-(piperazin-1-yl)phenyl)pyrazolo[1,5-a]pyrimidin-3-yl)quinoline hydrochloride (LDN193189), 6-[4-[2-(1-Piperidinyl)ethoxy]phenyl]-3-(4-pyridinyl)-pyrazolo[1,5-a]pyrimidine dihydrochloride (Dorsomorphin), 4-[6-[4-(1-Methylethoxy)phenyl]pyrazolo[1,5-a]pyrimidin-3-yl]-quinoline (DMH1), 4-[6-[4-[2-(4-Morpholinyl)ethoxy]phenyl]pyrazolo[1,5-a]pyrimidin-3-yl]quinoline (DMH-2), and 5-[6-(4-Methoxyphenyl)pyrazolo[1,5-a]pyrimidin-3-yl]quinoline (ML 347).

### TGFβ Pathway Inhibitors

Transforming growth factor beta (TGFβ) is a secreted protein that controls proliferation, cellular differentiation, and other functions in most cells. It is a type of cytokine which plays a role in immunity, cancer, bronchial asthma, lung fibrosis, heart disease, diabetes, and multiple sclerosis. TGF-β exists in at least three isoforms called TGF-β1, TGF-β2 and TGF-β3. The TGF-β family is part of a superfamily of proteins known as the transforming growth factor beta superfamily, which includes inhibins, activin, anti-müllerian hormone, bone morphogenetic protein, decapentaplegic and Vg-1.

TGFβ pathway inhibitors (also referred to herein as TGFβ inhibitors) may include any inhibitors of TGFβ signaling in general. For example, the TGFβ inhibitor is 4-[4-(1,3-benzodioxol-5-yl)-5-(2-pyridinyl)-1H-imidazol-2-yl]benzamide (SB431542), 6-[2-(1,1-Dimethylethyl)-5-(6-methyl-2-pyridinyl)-1H-imidazol-4-yl]quinoxaline (SB525334), 2-(5- Benzo[1,3]dioxol-5-yl-2-ieri-butyl-3H-imidazol-4-yl)-6-methylpyridine hydrochloride hydrate (SB- 505124), 4-(5-Benzol[l,3]dioxol- 5-yl-4-pyridin-2-yl-lH-imidazol-2-yl)-benzamide hydrate, 4-[4-(l,3-Benzodioxol-5-yl)-5-(2- pyridinyl)-lH-imidazol-2-yl]-benzamide hydrate, left-right determination factor (Lefty), 3-(6-Methyl-2-pyridinyl)-N-phenyl-4-(4-quinolinyl)-1H-pyrazole-1-carbothioamide (A 83-01), 4-[4-(2,3-Dihydro-1,4-benzodioxin-6-yl)-5-(2-pyridinyl)-1H-imidazol-2-yl]benzamide (D 4476), 4-[4-[3-(2-Pyridinyl)-1H-pyrazol-4-yl]-2-pyridinyl]-N-(tetrahydro-2H-pyran-4-yl)-benzamide (GW 788388), 4-[3-(2-Pyridinyl)-1H-pyrazol-4-yl]-quinoline (LY 364847), 4-[2-Fluoro-5-[3-(6-methyl-2-pyridinyl)-1H-pyrazol-4-yl]phenyl]-1H-pyrazole-1-ethanol (R 268712) or 2-(3-(6-Methylpyridine-2-yl)-1H-pyrazol-4-yl)-1,5-naphthyridine (RepSox).

### MEK Inhibitors

A MEK inhibitor is a chemical or drug that inhibits the mitogen-activated protein kinase enzymes MEK1 or MEK2. They can be used to affect the MAPK/ERK pathway. For example, MEK inhibitors include N-[(2R)-2,3-Dihydroxypropoxy]-3,4-difluoro-2-[(2-fluoro-4-iodophenyl)amino]- benzamide (PD0325901), N-[3-[3-cyclopropyl-5-(2-fluoro-4-iodoanilino)-6,8-dimethyl-2,4,7-trioxopyrido[4,3-d]pyrimidin-1-yl]phenyl]acetamide (GSK1120212), 6-(4-bromo-2-fluoroanilino)-7-fluoro-N-(2-hydroxyethoxy)-3-methylbenzimidazole-5-carboxamide (MEK162), N-[3,4-difluoro-2-(2-fluoro-4-iodoanilino)-6-methoxyphenyl]-1-(2,3-dihydroxypropyl)cyclopropane-1-sulfonamide (RDEA119), and 6-(4-bromo-2-chloroanilino)-7-fluoro-N-(2-hydroxyethoxy)-3-methylbenzimidazole-5-carboxamide (AZD6244).

### Gamma-secretase inhibitors

Gamma secretase is a multi-subunit protease complex, itself an integral membrane protein, that cleaves single-pass transmembrane proteins at residues within the transmembrane domain. Proteases of this type are known as intramembrane proteases. The most well-known substrate of gamma secretase is amyloid precursor protein, a large integral membrane protein that, when cleaved by both gamma and beta secretase, produces a short amino acid peptide called amyloid beta whose abnormally folded fibrillar form is the primary component of amyloid plaques found in the brains of Alzheimer's disease patients.

Gamma secretase inhibitors herein refer to γ-secretase inhibitors in general. For example, γ-secretase inhibitors include, but are not limited to *N*-[(3,5-Difluorophenyl)acetyl]-L-alanyl-2-phenyl]glycine-1,1-dimethylethyl ester (DAPT), 5-Chloro-*N*-[(1*S*)-3,3,3-trifluoro-1-(hydroxymethyl)-2-(trifluoromethyl)propyl]-2-thiophenesulfonamide (Begacestat), MDL-28170,3,5-Bis(4-nitrophenoxy)benzoic acid (Compound W), 7-Amino-4-chloro-3-methoxy-1*H*-2-benzopyran (JLK6), (5*S*)-(*tert-*Butoxycarbonylamino)-6-phenyl-(4*R*)-hydroxy-(2*R*)-benzylhexanoyl)-L-leucy-L-phenylalaninamide (L-685,485), (*R*)-2-Fluoro-α-methyl[1,1'-biphenyl]-4-acetic acid ((*R*)-Flurbiprofen; Flurizan), *N*-[(1*S*)-2-[[(7*S*)-6,7-Dihydro-5-methyl-6-oxo-5H-dibenz[b,d]azepin-7-yl]amino]-1-methyl-2-oxoethyl]-3,5-difluorobenzeneacetamide (Dibenzazepine; DBZ), *N-*[*cis*-4-[(4-Chlorophenyl)sulfonyl]-4-(2,5-difluorophenyl)cyclohexyl]-1,1,1-trifluoromethanesulfonamide (MRK560), (2*S*)-2-[[(2*S*)-6,8-Difluoro-1,2,3,4-tetrahydro-2-naphthalenyl]amino]*-N*-[1-[2-[(2,2-dimethylpropyl)amino]-1,1-dimethylethyl]-1*H*-imidazol-4-yl]pentanamide dihydrobromide (PF3084014 hydrobromide) and 2-[(1*R*)-1-[[(4-Chlorophenyl)sulfonyl](2,5-difluorophenyl)amino]ethyl-5-fluorobenzenebutanoic acid (BMS299897).

### Cyclin Dependent Kinase Inhibitors

Cyclin dependent kinases (CDKs) are a family of sugar kinases first discovered for their role in regulating the cell cycle. They are also involved in regulating transcription, mRNA processing, and the differentiation of nerve cells. In many human cancers, CDKs are overactive or CDK-inhibiting proteins are not functional. CDK inhibitors may be CDK1, CDK2, CDK3, CDK4, CDK5, CDK6, CDK7, CDK8, and/or CDK9 inhibitors. In particular aspects, the CDK inhibitor is a CDK4/6 inhibitor.

CDK inhibitors may include, but are not limited to, Palbociclib (PD-0332991) HCl, Roscovitine (Seliciclib, CYC202), SNS-032 (BMS-387032), Dinaciclib (SCH727965), Flavopiridol (Alvocidib), MSC2530818, JNJ-7706621, AZD5438, MK-8776 (SCH 900776), PHA-793887, BS-181 HCl, A-674563, abemaciclib (LY2835219), BMS-265246, PHA-767491, or Milciclib (PHA-848125).

### bFGF Inhibitors

Basic fibroblast growth factor (also known as bFGF, FGF2 or FGF-β) is a member of the fibroblast growth factor family. bFGF is present in basement membranes and in the subendothelial extracellular matrix of blood vessels. In addition, bFGF is a common component of human ESC culture medium in which it is necessary for the cells to remain in an undifferentiated state.

bFGF inhibitors herein refer to bFGF inhibitors in general. For example, bFGF inhibitors include, but are not limited to N-[2-[[4-(Diethylamino)butyl]amino-6-(3,5-dimethoxyphenyl)pyrido[2,3-d]pyrimidin-7-yl]-N'-(l,l-dimethylethyl)urea (PD173074), 2-(2-Amino-3-methoxyphenyl)-4H-l-benzopyran-4-one (PD 98059), l-tert-Butyl-3-[6-(2,6-dichlorophenyl)-2-[[4-(diethylamino)butyl]amino]pyrido[2,3-d]pyrimidin-7-yl]urea (PD161570), 6-(2,6-Dichlorophenyl)-2-[[4-[2-(diethylamino)ethoxy]phenyl]amino]-8-methyl-pyrido[2,3- d]pyrimidin-7(8H)-one dihydrochloride hydrate (PD166285), N-[2-Amino-6-(3,5-dimethoxyphenyl)pyrido[2,3-d]pyrimidin-7-yl]-N'-(1,1-dimethylethyl)-urea (PD166866), and MK-2206.

### IV. RPE-PR/PRP Dual Cell Aggregate Culture

In particular aspects, the RPE may be polarized, non-polarized, mature, immature, or a combination thereof. The RPE may be cultured in a media comprising taurine, hydrocortisone, and taurine, such as RPE-MM media described herein. In particular aspects, the media is serum-free or defined media and may comprise knockout serum replacement.

In some aspects, the RPE may be cultured to generate polarized RPE that are positive for Bestrophin and/or ZO1. The polarized RPE may be positive for PRE65 and/or Ezrin. The RPE may be derived from hiPSCs and may be mature, such as RPE positive for PMEL17, TYRP1, and CRALBP. The mature RPE, such as day 42 RPE by the methods described above, may be cultured directly or thawed if previously cryopreserved. The RPE can then be cultured for a period of time sufficient to polarize the RPE, such as 1 week, 2 weeks, 3 weeks, 4 weeks, 5 weeks, or more. In particular, the RPE may be cultured for 20, 21, 22, 23, 24, 25, 26, 27, 28, 29, or 30 days for polarization.

The PRP may be immature PRPs that can be fated to become either rods or cones. Specifically, the PRPs may be derived from the hybrid differentiation method described herein. The PRPs may be directly added to the RPE culture from culture or cryopreservation, or they may be re-plated and cultured prior to seeding with the RPE. In particular embodiments, the PRPs are derived from hiPSCs and not from sorting of organoids. The PRPs that are seeded may be essentially single cells free of aggregates. In other aspects, the PRPs may be added as aggregates. In particular aspects, the media is serum-free or defined media and may comprise knockout serum replacement.

RPE may be thawed in RPE-MM and counted using a ViCELL XR. The PRP may be thawed in RMN. If thawed as aggregates, the PRP may dissociated with TrypLE. The RPE and PRP may be combined at a desired cell ratio and cell number in RPE-MM and allowed to form aggregates, such as by being placed in a ULA T-75 flask on a belly dancer at 16-18 RPM to permit aggregate formation. Alternately, aggregates could be formed in a PBS mini vessel. The ROCK inhibitor Y-27632 is added to the culture for aggregate formation. The RPE may be cultured at a density of about 1 million cells/mL to about 10 million cells/mL, such as 2 million cells/mL, 3 million cells/mL, 4 million cells/mL, 5 million cells/mL, 6 million cells/mL, 7 million cells/mL, 8 million cells/mL, 9 million cells/mL or more. The ratio of the thawed PRP to RPE in the dual culture may be 500:1, 450:1, 400:1, 350:1, 300:1, 250:1, 200:1, 150:1, 100:1, or 50:1.

In one method, PRP and RPE may be combined as single-cell suspensions and permitted to form aggregates in RPE-MM with Y-27632. In another method, a single-cell suspension of RPE may be combined with PR/PRP aggregates in RPE-MM with Y-27632.

In some aspects, a ROCK inhibitor, an ECM protein, and/or prostaglandin E2 (PGE2) may be added to the RPE culture prior to the addition of the PRPs. The ROCK inhibitor is Y-27632. The ECM protein may be laminin, collagen I, collagen IV, fibronectin or vitronectin, particularly laminin-521. The ROCK inhibitor, ECM protein or PGE2 may be added at least 10 minutes, such as 30 minutes, 60 minutes, or 90 minutes, prior to the addition of the PRPs.

### V. Use of PRP:RPE Dual Cell Aggregates

Certain aspects provide a PRP:RPE dual cell aggregate culture which can be used for a number of important research purposes. A cryopreserved dual cell aggregate culture may be used as a therapeutic, such as a cell replacement therapy. The dual cell aggregate culture may be used for drug or toxin testing, as an in vitro model to study the biology of the retina, or a model with diseased cell lines that could be used for high-throughput drug screening. The present model system could be used for high-throughput experiments instead of animal studies with relevant RPE/photoreceptor interaction.

The PRP:RPE dual cell aggregate culture may be used for transplantation such as cell rescue therapy or whole tissue replacement therapy. Certain embodiments can provide use of PRP:RPE dual cell aggregate culture to enhance ocular tissue maintenance and repair for any condition in need thereof, including retinal degeneration or significant injury. Retinal degeneration may be associated with age-related macular degeneration (AMD), inherited macular degenerations, Stargardt's macular dystrophy, Best disease, choroideremia, inherited retinal degenerations (including retinitis pigmentosa, cone/rod and rod/cone dystrophies), diabetic retinopathy, retinal vascular disease, damage caused by retinopathy pf prematurity (ROP), viral infection of the eye, and other retinal/ocular diseases or injuries/trauma.

In another aspect, the disclosure provides a method of treatment of an individual in need thereof, comprising transplanting a composition comprising the PRP:RPE dual cell aggregate culture to said individual. Said composition may be administered to the eye, subretinal space, or intravenously. Such individuals may have macular degeneration including age- related macular degeneration, and such macular degeneration may be early or late stage. Such individuals may have inherited macular or retinal degenerations such as retinitis pigmentosa, cone/rod or rod/cone dystrophy, Stargardt's disease, Best disease, choroideremia, retinal dysplasia, retinal degeneration, diabetic retinopathy, congenital retinal dystrophy, Leber congenital amaurosis, retinal detachment, damage caused by retinopathy of prematurity (ROP), or other retinal trauma or injury.

The PRP:RPE dual cell aggregate culture produced by the methods disclosed herein may be used in any methods and applications currently known in the art for RPE or photoreceptor cells. For example, a method of assessing a compound may be provided, comprising assaying a pharmacological or toxicological property of the compound on the PRP:RPE dual cell aggregate culture. There may also be provided a method of assessing a compound for an effect on a PRP:RPE dual cell aggregate culture, comprising: a) contacting the PRP:RPE dual cell aggregate culture provided herein with the compound; and b) assaying an effect of the compound on the PRP:RPE dual cell aggregate culture. The dual cell aggregate of the present embodiments may also be used to produce retinal disease models to study pathophysiology and for drug screening.

The PRP:RPE dual cell aggregate culture can be used commercially to screen for factors (such as solvents, small molecule drugs, peptides, oligonucleotides) or environmental conditions (such as culture conditions or manipulation) that affect the characteristics of such cells and their various progeny. For example, test compounds may be chemical compounds, small molecules, polypeptides, growth factors, cytokines, or other biological agents. The PRP:RPE dual cell aggregate culture could be used to detect or screen for toxicity, retinoid recycling, phagocytosis, oxidative stress markers, or cell death.

In one embodiment, a method includes contacting a PRP:RPE dual cell aggregate culture with a test agent and determining if the test agent modulates activity or function of RPE or PRP cells within the population. In some applications, screening assays are used for the identification of agents that modulate cell proliferation, alter cell differentiation, or affect cell viability. Screening assays may be performed *in vitro* or *in vivo.* Methods of screening and identifying ocular agents or RPE:PRP agents include those suitable for high-throughput screening. For example, PRP:RPE dual cell aggregate culture can be positioned or placed on a culture dish, flask, roller bottle or plate (*e.g.,* a single multi-well dish or dish such as 8, 16, 32, 64, 96, 384 and 1536 multi-well plate or dish), optionally at defined locations, for identification of potentially therapeutic molecules. Libraries that can be screened include, for example, small molecule libraries, siRNA libraries, and adenoviral transfection vector libraries.

Other screening applications relate to the testing of pharmaceutical compounds for their effect on retinal tissue maintenance or repair. Screening may be done either because the compound is designed to have a pharmacological effect on the cells, or because a compound designed to have effects elsewhere may have unintended side effects on cells of this tissue type.

To determine suitability of cell compositions for therapeutics administration, the cells can first be tested in a suitable animal model, such as pigs. In one aspect, the PRP:RPE dual cell aggregate culture is evaluated for its ability to survive and maintain phenotype *in vivo.* The compositions are transplanted to immunodeficient animals (*e.g.,* nude mice or rats or animals rendered immunodeficient chemically or by irradiation). Tissues are harvested after a period of growth and assessed as to whether the pluripotent stem cell-derived cells are still present.

The PRP:RPE dual cell aggregate culture described herein, or a pharmaceutical composition including the culture, can be used for the manufacture of a medicament to treat a condition in a patient in need thereof. The PRP or RPE cells can be previously cryopreserved. In certain aspects, the disclosed PRP or RPE cells are derived from iPSCs, and thus can be used to provide "personalized medicine" for patients with eye diseases. In some embodiments, somatic cells obtained from patients can be genetically engineered to correct the disease-causing mutation, differentiated into PRP or RPE, and engineered to form the PRP:RPE dual cell aggregate culture. This tissue can be used to replace the endogenous degenerated PRP and RPE of the same patient. Alternatively, iPSCs generated from a healthy donor or from HLA homozygous "super-donors" can be used.

Various eye conditions may be treated or prevented by the introduction of the PRP:RPE dual cell aggregate culture obtained using the methods disclosed herein. The conditions include retinal diseases or disorders generally associated with retinal dysfunction or degradation, retinal injury, and/or loss of retinal pigment epithelium and/or photoreceptors. Conditions that can be treated include, without limitation, degenerative diseases of the retina, such as Stargardt's macular dystrophy, retinitis pigmentosa, rod/cone and cone/rod dystrophies, macular degeneration (such as age-related macular degeneration, myopic macular degeneration, or other acquired or inherited macular degenerations), retinal damage caused by retinopathy of prematurity (ROP) and diabetic retinopathy. Additional conditions include Lebers congenital amaurosis, hereditary or acquired macular or retinal degenerations, Best disease, retinal detachment, gyrate atrophy, choroideremia, pattern dystrophy, other dystrophies of photoreceptor cells, and retinal damage due to damage caused by any one of photic, laser, inflammatory, infectious, radiation, neovascular or traumatic injury. In certain embodiments, methods are provided for treating or preventing a condition characterized by retinal degeneration, comprising administering to a subject in need thereof an effective amount of a composition comprising the PRP:RPE dual cell aggregate culture. These methods can include selecting a subject with one or more of these conditions, and administering a therapeutically effective amount of the PRP:RPE dual cell aggregate culture sufficient to treat the condition and/or ameliorate symptoms of the condition. The PRP:RPE dual cell aggregate culture may be transplanted in various formats. For example, the PRP:RPE dual cell aggregate culture may be introduced into the target site adhered onto a matrix, extracellular matrix or substrate such as a biodegradable polymer.

Advantageously, the pharmaceutical preparations of the present disclosure may be used to compensate for a lack or diminution of RPE and/or photoreceptor cell function. Examples of retinal dysfunction that can be treated by the retinal cell populations and methods of the invention include but are not limited to: photoreceptor degeneration (as occurs in, *e.g.,* retinitis pigmentosa, cone dystrophies, cone-rod and/or rod-cone dystrophies, and inherited and age-related or myopic macular degeneration); retinal detachment and retinal trauma; photic lesions caused by laser or sunlight; a macular hole; a macular edema; night blindness and color blindness; ischemic retinopathy as caused by diabetes or vascular occlusion; retinopathy/retinal damage due to prematurity/premature birth; infectious conditions, such as CMV, retinitis and toxoplasmosis; inflammatory conditions, such as the uveitidies; retinal damage caused by tumors, such as retinoblastoma and ocular melanoma.

In one aspect, the cells can treat or alleviate the symptoms of inherited retinal degenerations such as retinitis pigmentosa, cone/rod or rod/cone dystrophies, Leber congenital amaurosis, or retinal injuries/trauma/damage in a patient in need of the treatment. In another aspect, the cells can treat or alleviate the symptoms of acquired or inherited forms of macular degeneration, such as age-related macular degeneration (wet or dry), Stargardt's disease, Best disease, myopic macular degeneration or the like, in a patient in need of this treatment. For all of these treatments, the cells can be autologous or allogeneic to the patient. In a further aspect, the cells of the present disclosure can be administered in combination with other treatments.

In some embodiments, the PRP:RPE dual cell aggregate culture can be used for autologous grafts to those subjects suitable for receiving regenerative medicine. The PRP:RPE dual cell aggregate culture may be transplanted in combination with other retinal cells. Transplantation of the PRP:RPE dual cell aggregate culture produced by the disclosed methods can be performed by various techniques known in the art. In accordance with one embodiment, the transplantation is performed via a pars plana surgical approach followed by delivery of the cells through a small retinal opening into the sub-retinal space or by direct injection. The PRP:RPE dual cell aggregate culture can be introduced into the target site as adhered onto a matrix, such as extracellular matrix, or provided on substrate such as a biodegradable polymer.

The PRP:RPE dual cell aggregate culture can be used to generate neurosensory retinal structures. These structures can be used for drug screening, as models for diseases, or as or in a pharmaceutical preparation. In the latter case, the pharmaceutical preparation can be an RPE-photoreceptor graft, which may be disposed on a biocompatible solid support or matrix (preferably a bioresorbable matrix or support) that can be implanted like a "patch".

To further illustrate, the biocompatible support for the cells can be a biodegradable synthetic, such as polyester, film support for RPE. The biodegradable polyester can be any biodegradable polyester suitable for use as a substrate or scaffold for supporting the proliferation and differentiation of retinal progenitor cells. The polyester should be capable of forming a thin film, preferably a micro-textured film, and should be biodegradable if used for tissue or cell transplantation. Suitable biodegradable polyesters for use in the invention include polylactic acid (PLA), polylactides, polyhydroxyalkanoates, both homopolymers and copolymers, such as polyhydoxybutyrate (PHB), polyhydroxybutyrate co-hydroxyvalerate (PHBV), polyhydroxybutyrate co-hydroxyhexanote (PHBHx), polyhydroxybutyrate co-hydroxyoctonoate (PHBO) and polyhydroxybutyrate co-hydroxyoctadecanoate (PHBOd), polycaprolactone (PCL), polyesteramide (PEA), aliphatic copolyesters, such as polybutylene succinate (PBS) and polybutylene succinate/adipate (PBSA), aromatic copolyesters. Both high and low molecular weight polyesters, substituted and unsubstituted polyester, block, branched or random, and polyester mixtures and blends can be used.

Pharmaceutical compositions of the PRP:RPE dual cell aggregate culture produced by the methods disclosed herein are also provided. These compositions can include at least about 1 x 10³ cells, about 1 x 10⁴ cells, about 1 x 10⁵ cells, about 1 x 10⁶ cells, about 1 x 10⁷ cells, about 1 x 10⁸ cells, or about 1 x 10⁹ cells. In certain embodiments, the compositions are substantially purified (with respect to non-PRP:RPE cells) preparations comprising differentiated PRP:RPE cells produced by the methods disclosed herein. Compositions are also provided that include a scaffold, such as a polymeric carrier and/or an extracellular matrix, and an effective amount of the PRP:RPE cells produced by the methods disclosed herein. The matrix material is generally physiologically acceptable and suitable for use in *in* vivo applications. For example, the physiologically acceptable materials include, but are not limited to, solid matrix materials that are absorbable and/or non-absorbable, such as small intestine submucosa (SIS), crosslinked or non-crosslinked alginate, hydrocolloid, foams, collagen gel, collagen sponge, polyglycolic acid (PGA) mesh, fleeces and bioadhesives.

Suitable polymeric carriers also include porous meshes or sponges formed of synthethic or natural polymers, as well as polymer solutions. For example, the matrix is a polymeric mesh or sponge, or a polymeric hydrogel. Natural polymers that can be used include proteins such as collagen, albumin, and fibrin; and polysaccharides such as alginate and polymers of hyaluronic acid. Synthetic polymers include both biodegradable and non-biodegradable polymers. For example, biodegradable polymers include polymers of hydroxy acids such as polyactic acid (PLA), polyglycolic acid (PGA) and polylactic acid-glycolic acid (PGLA), polyorthoesters, polyanhydrides, polyphosphazenes, and combinations thereof. Non-biodegradable polymers include polyacrylates, polymethacrylates, ethylene vinyl acetate, and polyvinyl alcohols.

Polymers that can form ionic or covalently crosslinked hydrogels which are malleable can be used. A hydrogel is a substance formed when an organic polymer (natural or synthetic) is cross- linked via covalent, ionic, or hydrogen bonds to create a three-dimensional open-lattice structure which entraps water molecules to form a gel. Examples of materials which can be used to form a hydrogel include polysaccharides such as alginate, polyphosphazines, and polyacrylates, which are crosslinked ionically, or block copolymers such as PLURON1CS^{™} or TETRON1CS^{™}, polyethylene oxide-polypropylene glycol block copolymers which are crosslinked by temperature or H, respectively. Other materials include proteins such as fibrin, polymers such as polyvinylpyrrolidone, hyaluronic acid and collagen.

The pharmaceutical compositions can be optionally packaged in a suitable container with written instructions for a desired purpose, such as the reconstitution of PRP cell function to improve a disease or abnormality of the retinal tissue. In some embodiments, the PRP cells produced by the disclosed methods may be used to replace degenerated photoreceptor cells of a subject in need therein.

### VI. Kits

In the present disclosure, a kit that can include, for example, one or more media and components for the production of PRP:RPE dual cell aggregate culture is provided. Such formulations may comprise a cocktail of retinal differentiation and/or trophic factors, in a form suitable for combining with photoreceptor precursor or photoreceptor cells. The reagent system may be packaged either in aqueous media or in lyophilized form, where appropriate. The container means of the kits will generally include at least one vial, test tube, flask, bottle, syringe or other container means, into which a component may be placed, and preferably, suitably aliquoted. Where there is more than one component in the kit, the kit also will generally contain a second, third or other additional container into which the additional components may be separately placed. However, various combinations of components may be comprised in a vial. The components of the kit may be provided as dried powder(s). When reagents and/or components are provided as a dry powder, the powder can be reconstituted by the addition of a suitable solvent. It is envisioned that the solvent may also be provided in another container means. The kits also will typically include a means for containing the kit component(s) in close confinement for commercial sale. Such containers may include injection or blow molded plastic containers into which the desired vials are retained. The kit can also include instructions for use, such as in printed or electronic format, such as digital format.

### VII. Examples

The following examples are included to demonstrate preferred embodiments of the invention. Subject-matter of examples not encompassed by the claims is disclosed for reference. It should be appreciated by those of skill in the art that the techniques disclosed in the examples which follow represent techniques discovered by the inventor to function well in the practice of the invention, and thus can be considered to constitute preferred modes for its practice.

### Example 1 - Dual Cell Aggregate Culture

To characterize a dual cell aggregate culture, RPE were thawed in RPE-MM and PRP were thawed in RMN. If PRP were thawed as aggregates, either all aggregates or a sample of aggregates were TrypLE dissociated. Both cell types were counted using a ViCELL XR. RPE and PRP were combined at a desired cell ratio and cell number in RPE-MM and placed in a ULA T-75 flask on a belly dancer at 16-18 RPM to permit aggregate formation. Alternately, aggregates could be formed in a PBS mini vessel. To test *in vivo* function, some aggregates were transplanted into the subretinal space of a rat retina two days after aggregate formation. Surgery was performed to analyze the retinas. Aggregates or rat retinas were cryosectioned and analyzed by immunocytochemistry.

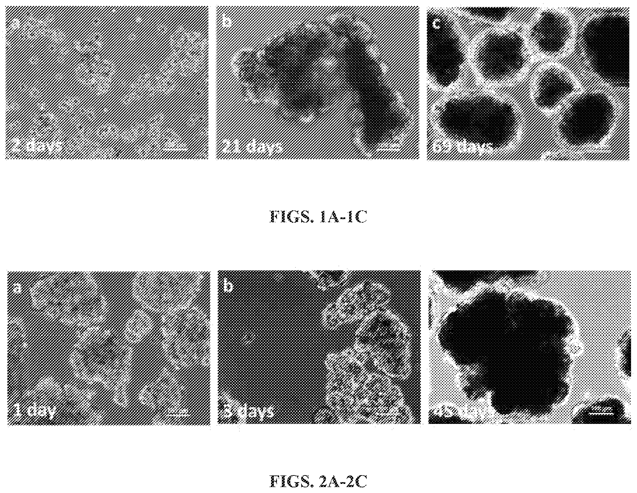

**Dual cell aggregate formation without Y-27632 in thaw medium:** As a first attempt to co-culture RPE and PRP as aggregates, RPE and PRP were thawed as single cells at a ratio of 1 RPE : 3 PRP. Phase contrast images were captured at various time points (FIG. 1). 2 days after thaw, there were several single cells that did not incorporate into aggregates, and based on qualitative evaluation of cell size, these cells appeared to be RPE. However, after 21 and 69 days, aggregates had formed and appeared to be fairly regular, though skewed to a high RPE ratio, relative to PRP. Based on this experiment, all future experiments used the ROCK inhibitor Y-27632 when thawing RPE and maintained a lower RPE:PRP ratio.

**Dual cell aggregate formation with Y-27632:** Next, RPE and PRP were thawed at a ratio of 1 RPE : 8 PRP in RPE-MM with Y-27632. In this thaw media, aggregates appeared to form more uniformly and efficiently (FIG. 2). With Y-27632 in the thaw medium, aggregate formation was qualitatively more efficient. However, RPE appeared to overgrow the aggregates by day 45.

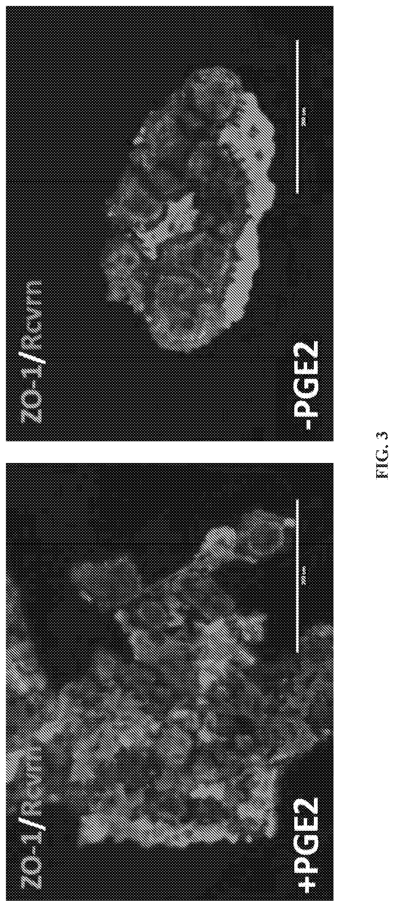

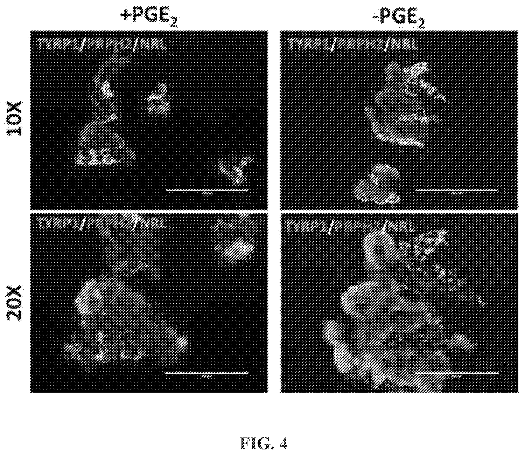

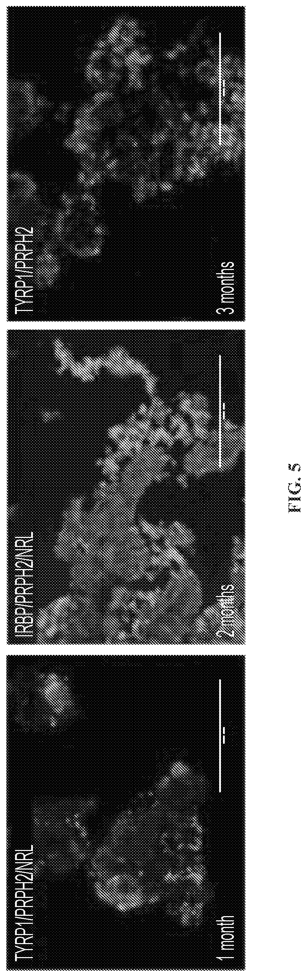

**Effect of PGE-2 on long-term dual cell aggregate culture:** During RPE culture, Prostaglandin E2 (PGE-2) is added to the culture medium from day 54 to day 68 of differentiation. PGE-2 is a hormone that stimulates RPE primary cilia formation and polarization, and its effect was tested on the dual cell aggregates (FIGS. 3-5). With this formulation of RPE and PRP, RPE generally appeared to form a majority of the aggregate, even after one month of culture. PRP did appear to mature after two months, indicated by the presence of PRPH2, an outer segment marker.

**Long-term dual cell aggregate evaluation *in vitro:*** For the next studies, an RPE:PRP ratio of was used 1:30 to form aggregates. To begin the study, two formulations were used. First, PRP and RPE were combined as single-cell suspensions and permitted to form aggregates in RPE-MM with Y-27632 (Condition A). Second, a single-cell suspension of RPE was combined with PRP aggregates in RPE-MM with Y-27632 (Condition B). Condition A was studied in detail *in vitro,* and both Condition A and Condition B were transplanted into rats for *in vivo* study.

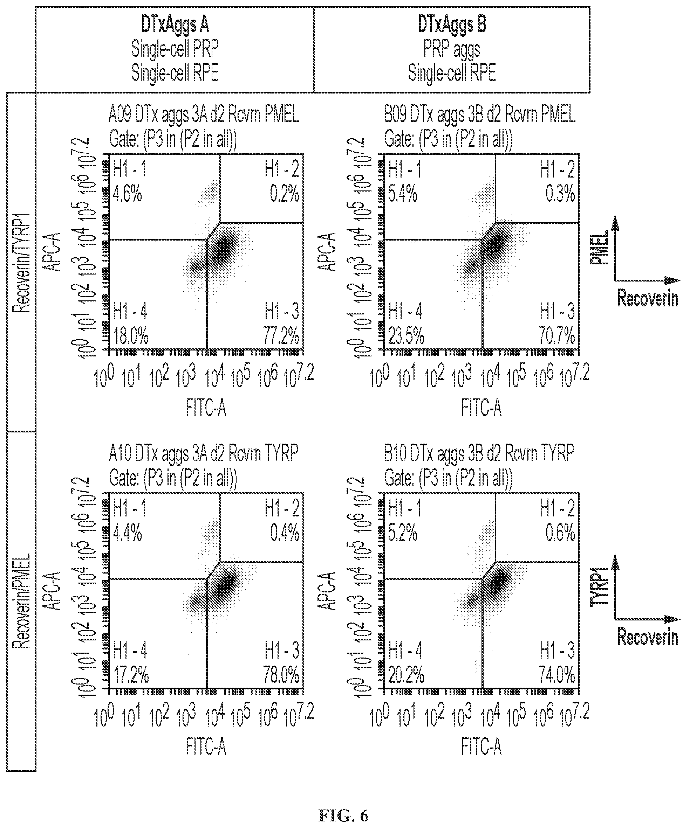

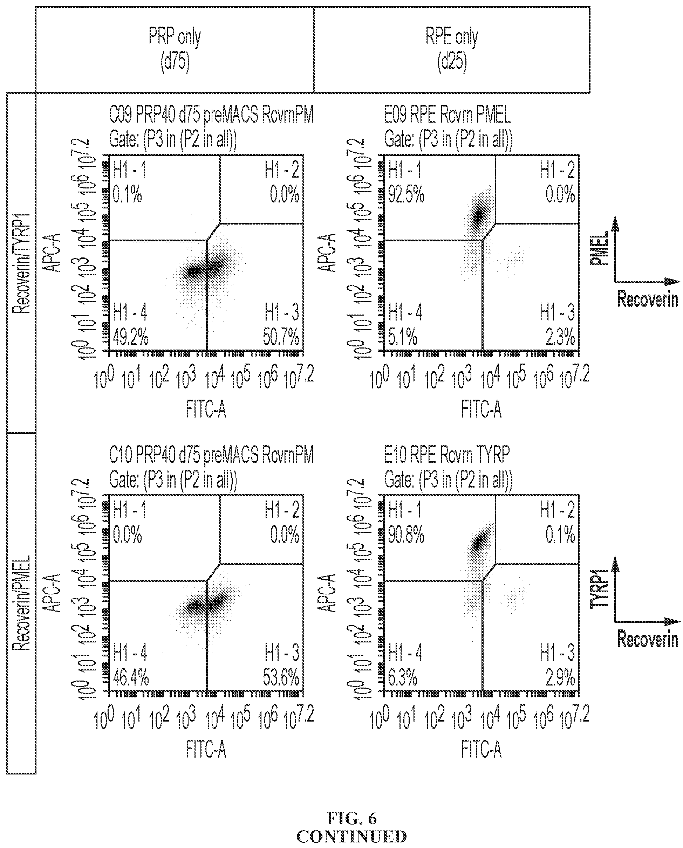

**Dual cell aggregate evaluation by flow cytometry:** First, the composition of aggregates was evaluated after two days by flow cytometry. Based on RPE and PRP marker comparison, a large fraction of the 2-day aggregates were PRP, though the measured proportion of RPE had increased from aggregate formation (FIG. 6). The RPE:PRP ratio appeared to be roughly equal between Condition A and Condition B.

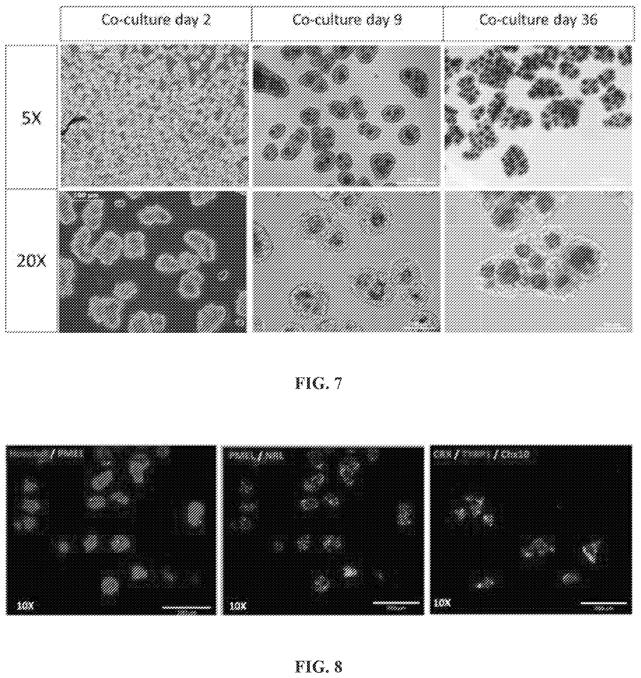

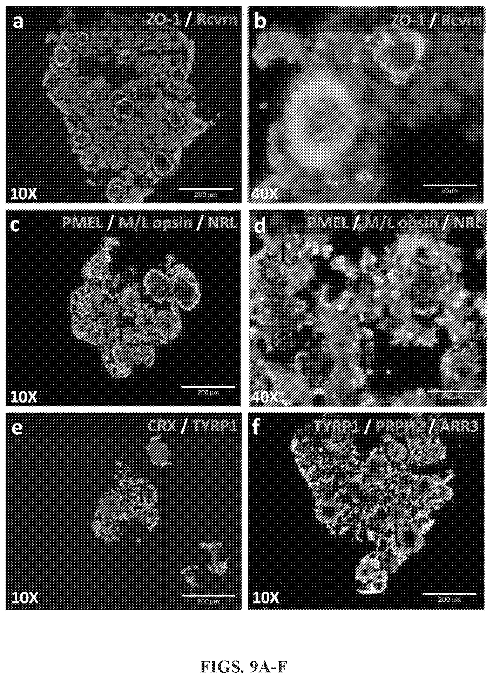

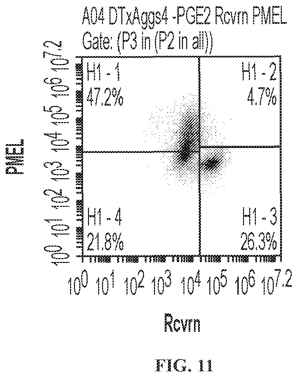

**Dual cell aggregate evaluation by immunocytochemistry:** Condition A (aggregate formation with single-cell suspensions of both RPE and PRP) was evaluated further *in vitro.* Phase contrast microscopy showed that aggregates were relatively uniform, but pigmented RPE was only visible after about 9 days of co-culture (FIG. 7). Aggregates appeared to fuse after extended culture. Immunocytochemistry of aggregates two days after aggregate formation confirmed that RPE is initially a small fraction of the co-culture (FIG. 8). After one month, both RPE and PRP were present, with pockets of RPE clusters apparent (FIG. 9). It is likely that multiple aggregates fused together to produce multiple RPE pockets in a single aggregate. After three months of culture *in vitro,* distinct regions of RPE and PRP were apparent, with pockets of RPE maturing and expressing RPE65 (FIG. 10). Immunocytochemistry results were confirmed by flow cytometry, with more RPE than PRP cells, and a small number of Ki67-positive proliferative cells that appeared to be immature RPE (FIG. 11).

**Dual cell aggregate transplantation into rat model:** Two formulations of dual cell aggregates were evaluated *in vivo* in the Royal College of Surgeons (RCS) rat. This rat has a mutation in the RPE gene MERTK, which leads to RPE dysfunction and subsequent photoreceptor death. PRP and RPE were combined as single-cell suspensions and permitted to form aggregates in RPE-MM with Y-27632 (Condition A). Separately, a single-cell suspension of RPE was combined with PRP aggregates in RPE-MM with Y-27632 (Condition B). Two days after aggregate formation, dual cell aggregates (both Condition A and Condition B) were transplanted into a rat model. Aggregate morphology before transplant is shown by phase contrast microscopy in FIG. 7 and by immunocytochemistry in FIG. 8.

**1 month *in vivo* results:** One month after surgery, the RPE and PRP generally appeared to have migrated to the correct retinal layer, though there were generally some RPE that had formed some small clusters in the neural retina layer. In condition A, both rod and cone photoreceptors were apparent. Condition B showed similar rod and cone distribution in the photoreceptors.

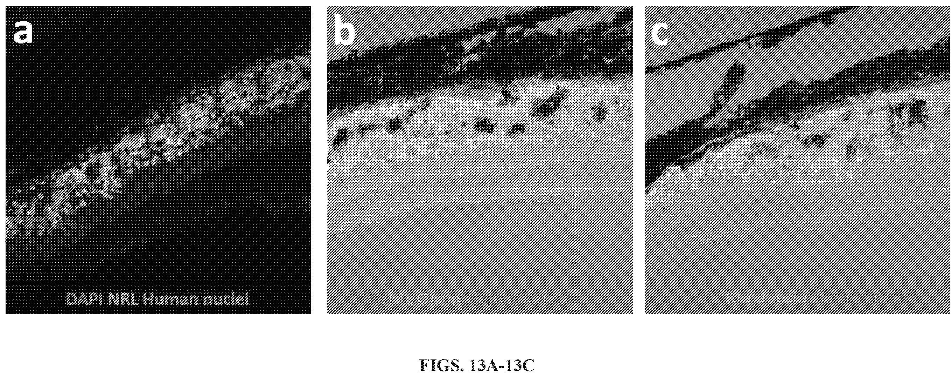

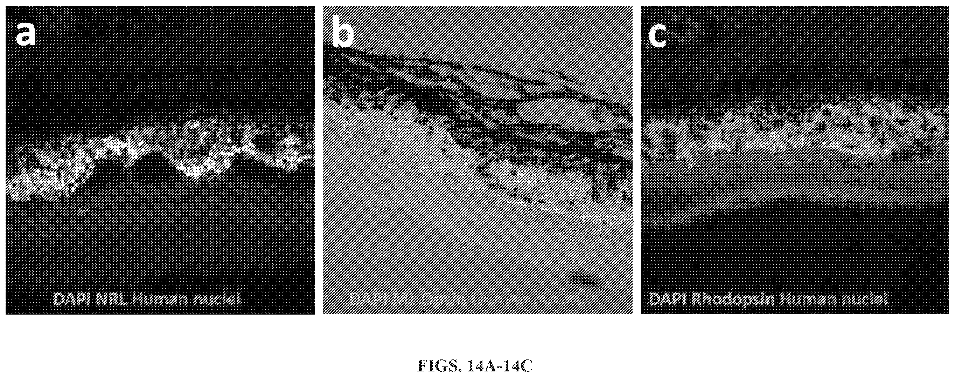

**2 month *in vivo* results:** After two months in Condition A, rod and cone maturation is apparent, and the photoreceptor layer generally appears consistent. However, small clusters of RPE remain in the photoreceptor layer, and there are also some proliferative cells (FIG. 13). In condition B, rod and cone maturation is also apparent, and some Ki67- and Pax6-positive cells are also present (FIG. 14). Thus, RPE and PRP cells were successfully combined in aggregate format. Iterative experiments show that RPE proliferates over time, and that a low RPE:PRP ratio may be desirable. Complex RPE:PRP interactions were apparent by immunocytochemistry, and the *in vitro* data show feasibility for modeling a retina in a dish. When dual cell aggregates were delivered into the subretinal space of the rat retina, RPE and PRP largely appeared to integrate into the correct retinal layer. However, there were consistently some clusters of RPE apparent in the photoreceptor layer, and proliferative cells were present. Together these studies suggest that dual cell aggregates are feasible for both *in vitro* modeling of a retina and potentially as a therapeutic.

### REFERENCES

Alexander et al., Proc. Nat. Acad. Sci. USA, 85:5092-5096,1988.
Barnea-Cramer et al. Sci Rep. 6:29784, 2016.
Bhutto and Lutty. Mol Aspects Med. 33(4):295-317, 2012.
Ercolani et al., J. Biol. Chem., 263:15335-15341,1988.
Hirami et al., Neurosci. Lett., 48: 126-131, 2009.
Hirami et al., Neurosci. Lett., 48: 126-131, 2009.
International Patent No. PCT/US2016/050543
International Patent No. PCT/US2016/050554
International Patent No. PCT/US2019/028557
International Publication No. WO 98/30679.
Karin et al. Cell, 36:371-379,1989.
Ludwig et al., Nat. Biotechnol., 24:185-187, 2006b.
Ludwig et al., Nat. Methods, 3:637-646, 2006a.
Macejak and Sarnow, Nature, 353:90-94, 1991.
Ng, Nuc. Acid Res., 17:601-615, 1989.
Oner. Turk J Ophthalmol. 48(1):33-8, 2018.
PCT Publication No. WO 2007/069666
PCT Publication No. WO 2014/121077.
Pelletier and Sonenberg, Nature, 334(6180):320-325, 1988.
Pennington and DeAngelis. Eye Vis (Lond). 3:34, 2016.
Richards et al., Cell, 37:263-272, 1984.
Sambrook and Russel, Molecular Cloning: A Laboratory Manual, 3rd Ed. Cold Spring Harbor Lab. Press, 2001.
Shimizu et al Cell Growth Differ 8: 1349-1358, 1997.
Strauss et al., Physiological Reviews, 85:845-881, 2005.
Strauss. Physiol Rev. 85(3):845-81, 2005.
Takahashi et al., Cell, 126, 663-676, 2006.
Takahashi et al., Cell, 131, 861-872, 2007.
Thomson and Marshall, Curr. Top. Dev. Biol., 38:133-165, 1998.
Thomson and Odorico, Trends Biotechnol., 18(2):53-57, 2000.
Thomson et al. Proc. Natl. Acad. Scie. USA, 92:7844-7848, 1995.
U.S. Patent Application No. 2002/0055144.
U.S. Patent Application No. 2002/0076747.
U.S. Patent Application No. 2009/0148425.
U.S. Patent Application No. 2009/0246875.
U.S. Patent Application No. 2010/0210014.
U.S. Patent Application No. 2012/0196360.
U.S. Patent Application No. 2012/0276636.
U.S. Patent No. 4,683,202.
U.S. Patent No. 5,556,954.
U.S. Patent No. 5,843,780.
U.S. Patent No. 5,925,565.
U.S. Patent No. 5,928,906.
U.S. Patent No. 5,935,819.
U.S. Patent No. 6,103,470.
U.S. Patent No. 6,200,806.
U.S. Patent No. 6,416,998.
U.S. Patent No. 6,833,269.
U.S. Patent No. 7,029,913.
U.S. Patent No. 7,442,548.
U.S. Patent No. 7,598,364.
U.S. Patent No. 7,682,828.
U.S. Patent No. 7,989,425.
U.S. Patent No. 8,058,065.
U.S. Patent No. 8,071,369.
U.S. Patent No. 8,129,187.
U.S. Patent No. 8,268,620.
U.S. Patent No. 8,278,620.
U.S. Patent No. 8,546,140.
U.S. Patent No. 8,741,648.
U.S. Patent Publication No. 2003/0211603.
U.S. Patent Publication No. 2010/0003757.
Wong et al. The Lancet Global Health. 2(2):e106-e1, 2014.
Yu et al., Science, 318: 1917-1920, 2007.
Zhao et al. Development. 144(8):1368-81, 2017.
Zhou et al. Development. 142(19):3294-306, 2015.

## Claims

1. An *in vitro* method for producing a dual cell aggregate composition comprising retinal pigment epithelium cells (RPE) and photoreceptor precursor cells (PRP), the method comprising seeding RPE and PRP in culture medium comprising the ROCK inhibitor Y-27632 and culturing for a period of time sufficient to produce the dual cell aggregate composition, wherein the ratio of PRP to RPE is 2:1 to 50:1 at the time of assembly of the dual cell aggregate composition,.

2. The method of claim 1, wherein
i) the ratio of PRP to RPE is about 10:1 to about 50:1 at the time of assembly of the dual cell aggregate composition; or
ii) the RPE and/or the PRP are derived from pluripotent stem cells (PSCs), wherein optionally the PSCs are induced pluripotent stem cells (iPSCs) or embryonic stem cells (ESCs), wherein optionally the iPSCs are human iPSCs (hiPSCs); or
iii) the PRP were not derived from organoids.

3. The method of claim 1 or 2, wherein
i) the RPE and/or the PRP have been previously cryopreserved, wherein optionally the cryopreserved RPE and/or PRP have been thawed and cultured for at least one week; or
ii) the PRP are thawed and directly seeded with the RPE; or
iii) the RPE and/or the PRP are from same donor.

4. The method of any one of claims 1 to 3, wherein
i) the RPE are mature RPE expressing Bestrophin-1 (BEST1) and/or ZO-1; or
ii) the RPE are immature RPE with essentially no expression of BEST1 and/or ZO-1; or
iii) the RPE are polarized; or
iv) the RPE are not polarized;
v) the RPE and PRP are seeded as essentially single-cell suspensions; or
vi) the RPE are seeded as an essentially single-cell suspension and the PRP are seeded as aggregates; or
vii) Y-27632 is added at a concentration of 10 µM; or
viii) the culture medium further comprises prostaglandin E2 (PGE-2); or
ix) the RPE were previously cultured in the presence of PGE-2; or
x) the PRP express PRPH2; or
xi) the PRP are rod-disposed.

5. The method of any one of claims 1-3, wherein
i) the PRP are cone-disposed; or
ii) the RPE and PRP are seeded at a density of 1 million cells/mL to 10 million cells/mL; or
iii) the RPE and PRP are seeded at a density of about 5 million cells/mL; or
iv) the RPE and/or the PRP have been previously cryopreserved; or
v) the culture medium further comprises taurine and hydrocortisone; or
vi) the culture medium further comprises triiodothyronine; or
vii) the culture medium is defined media or serum-free media; or
viii) the culture medium comprises serum replacement; or
ix) the culture medium is RPE-MM media.

6. The method of any one of claims 1-5, wherein
i) the culturing is for at least 10 days; or
ii) the culturing is for 2 weeks to one month; or
iii) the culturing is for at least two months; or
iv) the culture medium is exchanged at least once every five days; or
v) the culture medium is exchanged at least once every three days; or
vi) the culture medium is exchanged at least once every other day; or
vii) the method further comprises cryopreserving the dual cell aggregate composition.

7. The composition produced by the method of any one of claims 1-6 for use in a method for treating an ocular injury or disorder in a subject, the method comprising transplanting an effective amount of the composition to an eye of the subject.

8. The composition for use of claim 7, wherein
the composition further comprises hyaluronate; or
the composition comprises hyaluronate at a concentration of less than about 0.5%; or
the composition further comprises sodium bicarbonate, calcium chloride, potassium chloride, potassium phosphate monobasic, magnesium chloride, magnesium sulfate, sodium chloride, and/or sodium phosphate dibasic; or
the composition comprises 200,000 to 3,000,000 cells; or
the composition comprises about 700,000 cells.

9. The composition for use of claim 7, wherein
i) the subject is administered a dose of 200,000 to 3,000,000 of the RPE and PRP cells; or
ii) the subject is administered a dose of about 700,000 of the RPE and PRP cells; or
iii) the composition is transplanted to the subretinal space of the eye; or
iv) the ocular disorder is due to RPE dysfunction or photoreceptor dysfunction; and/or wherein the ocular disorder is age-related macular degeneration, retinitis pigmentosa, cone-rod dystrophies, or Leber congenital amaurosis; or
v) the composition produces both rod and cone photoreceptors in the eye of the subject.

10. *Ex vivo* use of the dual cell aggregate composition produced by the method of any one of claims 1-6 as a model retina.

11. An *in vitro* method of screening a compound comprising contacting one or more candidate compounds with the dual cell aggregate composition produced by the method of any one of claims 1-6 and detecting an effect on the RPE-PRP dual cell aggregates, wherein
i) the one or more candidate compounds are selected from the group consisting of a chemical compound, small molecule, polypeptide, growth factor, solvent, oligonucleotide, and cytokine; or
ii) detecting an effect comprises measuring cell proliferation, cell viability, cell death, drug toxicity, or retinal tissue maintenance or repair; or
iii) the method is performed in a high-throughput manner, wherein optionally the dual cell aggregate composition is placed in a multi-well culture plate.

12. An *in vitro* retina model comprising the dual cell aggregate composition produced by the method of any one of claims 1-6, wherein optionally the RPE and/or PRP are obtained from a disease cell line, wherein optionally the disease is an ocular disease, wherein optionally the ocular disease is age-related macular degeneration, retinitis pigmentosa, cone-rod dystrophies, or Leber congenital amaurosis.

## Patentansprüche

1. *In-vitro-Verfahren* zur Herstellung einer dualen Zellaggregatzusammensetzung, die Retinalpigmentepithelzellen (RPE) und Photorezeptor-Vorläuferzellen (PRP) umfasst, wobei das Verfahren das Ausbringen von RPE und PRP in ein Kulturmedium, das den ROCK-Inhibitor Y-27632 enthält, und das Kultivieren über einen Zeitraum umfasst, der ausreicht, um die duale Zellaggregatzusammensetzung herzustellen, wobei das Verhältnis von PRP zu RPE zum Zeitpunkt der Bildung der dualen Zellaggregatzusammensetzung 2:1 bis 50:1 beträgt.

2. Verfahren nach Anspruch 1, wobei
i) das Verhältnis von PRP zu RPE zum Zeitpunkt der Bildung der dualen Zellaggregatzusammensetzung etwa 10:1 bis etwa 50:1 beträgt; oder
ii) die RPE und/oder die PRP von pluripotenten Stammzellen (PSCs) stammen, wobei die PSCs optional induzierte pluripotente Stammzellen (iPSCs) oder embryonale Stammzellen (ESCs) sind, wobei die iPSCs optional menschliche iPSCs (hiPSCs) sind; oder
iii) die PRP nicht aus Organoiden stammen.

3. Verfahren nach Anspruch 1 oder 2, wobei
i) die RPE und/oder die PRP zuvor kryokonserviert wurden, wobei die kryokonservierten RPE und/oder PRP optional aufgetaut und mindestens eine Woche lang kultiviert wurden; oder
ii) die PRP aufgetaut und direkt mit den RPE ausgebracht werden; oder
iii) die RPE und/oder die PRP von demselben Spender stammen.

4. Verfahren nach einem der Ansprüche 1 bis 3, wobei
i) die RPE reife RPE sind, die Bestrophin-1 (BEST1) und/oder ZO-1 exprimieren; oder
ii) die RPE unreife RPE sind, die im Wesentlichen keine Expression von BEST1 und/oder ZO-1 aufweisen; oder
iii) die RPE polarisiert sind; oder
iv) die RPE nicht polarisiert sind;
v) die RPE und PRP als im Wesentlichen einzellige Suspensionen ausgebracht werden; oder
vi) die RPE als im Wesentlichen einzellige Suspension ausgebracht werden und die PRP als Aggregate ausgebracht werden; oder
vii) Y-27632 in einer Konzentration von 10 µM zugegeben wird; oder
viii) das Kulturmedium ferner Prostaglandin E2 (PGE-2) umfasst; oder
ix) die RPE zuvor in Gegenwart von PGE-2 kultiviert wurden; oder
x) die PRP PRPH2 exprimieren; oder
xi) die PRP stäbchen-prädisponiert sind.

5. Verfahren nach einem der Ansprüche 1 bis 3, wobei
i) die PRP zapfen-prädisponiert sind; oder
ii) die RPE und die PRP mit einer Dichte von 1 Million Zellen/ml bis 10 Millionen Zellen/ml ausgebracht werden; oder
iii) die RPE und die PRP mit einer Dichte von etwa 5 Millionen Zellen/ml ausgebracht werden; oder
iv) die RPE und/oder die PRP zuvor kryokonserviert wurden; oder
v) das Kulturmedium ferner Taurin und Hydrocortison umfasst; oder
vi) das Kulturmedium ferner Trijodthyronin umfasst; oder
vii) das Kulturmedium ein definiertes Medium oder ein serumfreies Medium ist; oder
viii) das Kulturmedium einen Serumersatz umfasst; oder
ix) das Kulturmedium ein RPE-MM-Medium ist.

6. Verfahren nach einem der Ansprüche 1 bis 5, wobei
i) die Kultivierung mindestens 10 Tage dauert; oder
ii) die Kultivierung 2 Wochen bis einen Monat dauert; oder
iii) die Kultivierung mindestens zwei Monate dauert; oder
iv) das Kulturmedium mindestens einmal alle fünf Tage ausgetauscht wird; oder
v) das Kulturmedium mindestens einmal alle drei Tage ausgetauscht wird; oder
vi) das Kulturmedium mindestens jeden zweiten Tag ausgetauscht wird; oder
vii) das Verfahren ferner die Kryokonservierung der dualen Zellaggregatzusammensetzung umfasst.

7. Nach einem der Verfahren der Ansprüche 1 bis 6 hergestellte Zusammensetzung zur Verwendung in einem Verfahren zur Behandlung einer Augenverletzung oder -erkrankung in einem Wesen, wobei das Verfahren das Transplantieren einer wirksamen Menge der Zusammensetzung in ein Auge des Wesens umfasst.

8. Zusammensetzung zur Verwendung nach Anspruch 7, wobei
die Zusammensetzung ferner Hyaluronat umfasst; oder
die Zusammensetzung Hyaluronat in einer Konzentration von weniger als etwa 0,5 % umfasst; oder
die Zusammensetzung ferner Natriumbicarbonat, Calciumchlorid, Kaliumchlorid, einbasisches Kaliumphosphat, Magnesiumchlorid, Magnesiumsulfat, Natriumchlorid, und/oder zweibasisches Natriumphosphat umfasst; oder
die Zusammensetzung 200.000 bis 3.000.000 Zellen umfasst; oder
die Zusammensetzung etwa 700.000 Zellen umfasst.

9. Zusammensetzung zur Verwendung nach Anspruch 7, wobei
i) dem Wesen eine Dosis von 200.000 bis 3.000.000 der RPE- und PRP-Zellen verabreicht wird; oder
ii) dem Wesen eine Dosis von etwa 700.000 der RPE- und PRP-Zellen verabreicht wird; oder
iii) die Zusammensetzung in den subretinalen Raum des Auges transplantiert wird; oder
iv) die Augenerkrankung auf eine RPE-Dysfunktion oder eine Photorezeptor-Dysfunktion zurückzuführen ist; und/oder wobei die Augenerkrankung eine altersbedingte Makuladegeneration, Retinitis pigmentosa, Zapfen-Stäbchen-Dystrophien, oder eine Lebersche kongenitale Amaurose ist; oder
v) die Zusammensetzung sowohl Stäbchen- als auch Zapfen-Photorezeptoren im Auge des Wesens erzeugt.

10. Ex-vivo-Verwendung der nach einem der Verfahren nach den Ansprüchen 1 bis 6 hergestellten dualen Zellaggregatzusammensetzung als Modellretina.

11. *In-vitro-Verfahren* zum Screenen einer Verbindung, umfassend das Inkontaktbringen einer oder mehrerer Kandidatenverbindungen mit der nach einem der Verfahren nach den Ansprüchen 1 bis 6 hergestellten dualen Zellaggregatzusammensetzung und das Nachweisen einer Wirkung auf die RPE-PRP-dualen Zellaggregate, wobei
i) die eine oder mehrere Kandidatenverbindungen aus der Gruppe ausgewählt sind, die aus einer chemischen Verbindung, einer niedermolekularen Verbindung, einem Polypeptid, einem Wachstumsfaktor, einem Lösungsmittel, einem Oligonukleotid, und einem Zytokin besteht; oder
ii) das Nachweisen einer Wirkung das Messen der Zellproliferation, der Zellviabilität, des Zelltods, der Arzneimitteltoxizität oder der Erhaltung oder Reparatur von Netzhautgewebe umfasst; oder
iii) das Verfahren im Hochdurchsatzverfahren durchgeführt wird, wobei die duale Zellaggregatzusammensetzung optional in eine Multiwell-Kulturplatte gegeben wird.

12. In-vitro-Retinamodell, das die nach einem der Verfahren der Ansprüche 1 bis 6 hergestellte duale Zellaggregatzusammensetzung umfasst, wobei optional die RPE und/oder die PRP aus einer krankheitsspezifischen Zelllinie gewonnen werden, wobei optional die Krankheit eine Augenerkrankung ist, wobei optional die Augenerkrankung eine altersbedingte Makuladegeneration, Retinitis pigmentosa, Zapfen-Stäbchen-Dystrophien, oder eine Lebersche kongenitale Amaurose ist.

## Revendications

1. Procédé *in vitro* pour la production d'une composition d'agrégats cellulaires doubles comprenant des cellules de l'épithélium pigmentaire rétinien (RPE) et des cellules précurseurs des photorécepteurs (PRP), le procédé comprenant l'ensemencement de RPE et de PRP dans un milieu de culture comprenant l'inhibiteur de ROCK Y-27632 et la culture pendant une durée suffisante pour produire la composition d'agrégats cellulaires doubles, dans laquelle le rapport PRP/RPE est compris entre 2:1 et 50:1 au moment de l'assemblage de la composition d'agrégats cellulaires doubles.

2. Procédé selon la revendication 1, dans lequel
i) le rapport entre les PRP et les RPE est d'environ 10:1 à environ 50:1 au moment de l'assemblage de la composition d'agrégats cellulaires doubles ; ou
ii) le RPE et/ou le PRP sont dérivés de cellules souches pluripotentes (PSC), dans lequel, éventuellement, les PSC sont des cellules souches pluripotentes induites (iPSC) ou
des cellules souches embryonnaires (CSE), les iPSC étant éventuellement des iPSC humaines (hiPSC) ; ou
iii) les PRP ne sont pas dérivés d'organoïdes.

3. Procédé selon la revendication 1 ou 2, dans lequel
i) les RPE et/ou les PRP ont été préalablement cryoconservés, dans lequel éventuellement, les RPE et/ou les PRP cryoconservés ont été décongelés et cultivés pendant au moins une semaine ; ou
ii) les PRP sont décongelés et ensemencé directement avec les EPR ; ou
iii) les RPE et/ou les PRP proviennent du même donneur.

4. Procédé selon l'une quelconque des revendications 1 à 3, dans lequel
i) les RPE sont des RPE matures exprimant la bestrophine-1 (BEST1) et/ou la ZO-1 ; ou
ii) les RPE sont des RPE immatures ne présentant pratiquement aucune expression de BEST1 et/ou de ZO-1 ; ou
iii) les RPE sont polarisées ; ou
iv) les RPE ne sont pas polarisées ;
v) les RPE et les PRP sont ensemencées sous forme de suspensions essentiellement unicellulaires ; ou
vi) les RPE sont ensemencées sous forme de suspension essentiellement monocellulaire et les PRP sont ensemencées sous forme d'agrégats ; ou
vii) du Y-27632 est ajouté à une concentration de 10 µM ; ou
viii) le milieu de culture comprend en outre de la prostaglandine E2 (PGE-2) ; ou
ix) les RPE ont été préalablement cultivées en présence de PGE-2 ; ou
x) les PRP expriment la PRPH2 ; ou
xi) les PRP sont disposées en forme de bâtonnets.

5. Procédé selon l'une quelconque des revendications 1 à 3, dans lequel
i) les PRP sont disposées en forme de cône ; ou
ii) le RPE et les PRP sont ensemencés à une densité comprise entre 1 million de cellules/mL et 10 millions de cellules/mL ; ou
iii) l'EPR et le PRP sont ensemencés à une densité d'environ 5 millions de cellules/mL ; ou
iv) l'EPR et/ou le PRP ont été préalablement cryoconservés ; ou
v) le milieu de culture comprend en outre de la taurine et de l'hydrocortisone ; ou
vi) le milieu de culture comprend en outre de la triiodothyronine ; ou
vii) le milieu de culture est un milieu défini ou un milieu sans sérum ; ou
viii) le milieu de culture comprend un substitut de sérum ; ou
ix) le milieu de culture est un milieu RPE-MM.

6. Procédé selon l'une quelconque des revendications 1 à 5, dans lequel
i) la culture dure au moins 10 jours ; ou
ii) la culture dure de 2 semaines à un mois ; ou
iii) la culture dure au moins deux mois ; ou
iv) le milieu de culture est renouvelé au moins une fois tous les cinq jours ; ou
v) le milieu de culture est renouvelé au moins une fois tous les trois jours ; ou
vi) le milieu de culture est renouvelé au moins une fois tous les deux jours ; ou
vii) la méthode comprend en outre la cryoconservation de la composition d'agrégats cellulaires doubles.

7. La composition produite par le procédé selon l'une quelconque des revendications 1 à 6, destinée à être utilisée dans un
procédé de traitement d'une lésion ou d'un trouble oculaire chez un sujet, le procédé comprenant la transplantation d'une quantité efficace de la composition dans un œil du sujet.

8. La composition destinée à être utilisée selon la revendication 7, dans laquelle
la composition comprend en outre de l'hyaluronate ; ou
la composition comprend de l'hyaluronate à une concentration inférieure à environ 0,5 % ; ou
la composition comprend en outre du bicarbonate de sodium, du chlorure de calcium, du chlorure de potassium, du phosphate de potassium monobasique, du chlorure de magnésium, du sulfate de magnésium, du chlorure de sodium et/ou du phosphate de sodium dibasique ; ou la composition comprend 200 000 à 3 000 000 de cellules ; ou la composition comprend environ 700 000 cellules.

9. Composition selon la revendication 7, dans laquelle
i) le sujet reçoit une dose de 200 000 à 3 000 000 de RPE et
de cellules PRP ; ou
ii) on administre au sujet une dose d'environ 700 000 cellules RPE et PRP ; ou
iii) la composition est transplantée dans l'espace sous-rétinien de l'œil ; ou
iv) le trouble oculaire est dû à un dysfonctionnement de l'EPR ou à un dysfonctionnement des photorécepteurs ;
et/ou dans lequel le trouble oculaire est une dégénérescence maculaire liée à l'âge,
une rétinite pigmentaire, des dystrophies des cônes et des bâtonnets, ou une amaurose congénitale de Leber ; ou
v) la composition produit à la fois des photorécepteurs à cônes et à bâtonnets dans l'œil du sujet.

10. Utilisation *ex vivo* de la composition d'agrégats cellulaires doubles produite par le procédé de l'une des revendications 1 à 6 en tant que rétine modèle.

11. Procédé *in vitro* de criblage d'un composé comprenant la mise en contact d'un ou plusieurs composés candidats avec la composition d'agrégats cellulaires doubles produite par le procédé selon l'une quelconque des revendications 1 à 6 et la détection d'un effet sur les agrégats cellulaires doubles RPE-PRP, dans lequel
i) le ou les composés candidats sont choisis parmi le groupe constitué d'un composé chimique, d'une petite molécule, d'un polypeptide, d'un facteur de croissance, d'un solvant, d'un oligonucléotide et d'une cytokine ; ou
ii) la détection d'un effet comprend la mesure de la prolifération cellulaire, de la viabilité cellulaire, de la mort cellulaire, de la toxicité du médicament, ou du maintien ou de la réparation du tissu rétinien ; ou
iii) la méthode est réalisée selon un procédé à haut débit, dans lequel, éventuellement, la composition d'agrégats cellulaires doubles est placée dans une plaque de culture à puits multiples.

12. Modèle de rétine *in vitro* comprenant la composition d'agrégats cellulaires doubles produite par le procédé selon l'une quelconque des revendications 1 à 6, dans lequel, de manière facultative, les RPE et/ou les PRP sont obtenus à partir d'une lignée cellulaire pathologique, dans lequel, de manière facultative, la pathologie est une pathologie oculaire,
dans lequel, éventuellement, la maladie oculaire est une dégénérescence maculaire liée à l'âge, une rétinite pigmentaire, des dystrophies des cônes et des bâtonnets, ou de l'amaurose congénitale de Leber.
